(19) Europäisches Patentamt

European Patent Office

Office européen des brevets

(11) **EP 2 594 936 A2**

(12) **EUROPEAN PATENT APPLICATION**

(43) Date of publication:
**22.05.2013 Bulletin 2013/21**

(21) Application number: **12192525.9**

(22) Date of filing: **14.11.2012**

(51) Int Cl.:
*G01N 30/86* (2006.01)    *H01J 49/00* (2006.01)
*G01N 30/72* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.11.2011 US 201113300287**

(71) Applicant: **Thermo Finnigan LLC**
**San Jose, CA 95134 (US)**

(72) Inventor: **Wright, David A.**
**Livermore, CA 94550 (US)**

(74) Representative: **Horler, Philip John**
**Boult Wade Tennant**
**Verulam Gardens**
**70 Gray's Inn Road**
**London WC1X 8BT (GB)**

(54) **Methods and apparatus for identifying mass spectral isotope patterns**

(57)    A method for automatically identifying groups of related peaks generated in a chromatography - mass spectrometry experiment comprises automatically choosing a time window defining a region of interest for mass spectral data generated by the experiment; automatically constructing a plurality of extracted ion chromatograms (XICs) (m1-m10) for mass spectral peaks observed within the region of interest; automatically detecting and characterizing chromatogram peaks (p1-p10) within each XIC and automatically generating synthetic analytical fit peaks thereof; automatically discarding a subset of the synthetic analytical peaks which do not satisfy noise reduction rules; automatically performing a respective cross-correlation score calculation between each pair of synthetic analytical fit peaks; and automatically identifying groups of correlated peaks in at least one mass spectrum within the region of interest.

FIG. 15

**Description**

Field of the Invention

This invention relates to methods of analyzing data obtained from instrumental analysis techniques used in analytical chemistry and, in particular, to methods of automatically discriminating peaks of a related group of peaks - such as peaks representing an isotopic distribution - from noise, without input from or intervention of a user, in mass spectral data generated in LC/MS/MS analyses.

Background of the Invention

Mass spectrometry (MS) is an analytical technique to filter, detect, identify and/or measure compounds by the mass-to-charge ratios of ions formed from the compounds. The quantity of mass-to-charge ratio is commonly denoted by the symbol "m/z" in which "m" is ionic mass in units of Daltons and "z" is ionic charge in units of elementary charge, e. Thus, mass-to-charge ratios are appropriately measured in units of "Da/e". Mass spectrometry techniques generally include (1) ionization of compounds and optional fragmentation of the resulting ions so as to form fragment ions; and (2) detection and analysis of the mass-to-charge ratios of the ions and/or fragment ions and calculation of corresponding ionic masses. The compound may be ionized and detected by any suitable means. A "mass spectrometer" generally includes an ionizer and an ion detector.

The hybrid technique of liquid chromatography-mass spectrometry (LC/MS) is an extremely useful technique for detection, identification and (or) quantification of components of mixtures or of analytes within mixtures. This technique generally provides data in the form of a mass chromatogram, in which detected ion intensity (a measure of the number of detected ions) as measured by a mass spectrometer is given as a function of time. In the LC/MS technique, various separated chemical constituents elute from a chromatographic column as a function of time. As these constituents come off the column, they are submitted for mass analysis by a mass spectrometer. The mass spectrometer accordingly generates, in real time, detected relative ion abundance data for ions produced from each eluting analyte, in turn. Thus, such data is inherently three-dimensional, comprising the two independent variables of time and mass (more specifically, a mass-related variable, such as mass-to-charge ratio) and a measured dependent variable relating to ion abundance.

Generally, "liquid chromatography" (LC) means a process of selective retention of one or more components of a fluid solution as the fluid uniformly percolates through a column of a finely divided substance, or through capillary passageways. The retention results from the distribution of the components of the mixture between one or more stationary phases and the bulk fluid, (i.e., mobile phase), as this fluid moves relative to the stationary phase(s). "Liquid chromatography" includes, without limitation, reverse phase liquid chromatography (RPLC), high performance liquid chromatography (HPLC), ultra high performance liquid chromatography (UHPLC), supercritical fluid chromatography (SFC) and ion chromatography.

High-resolution mass spectrometer instruments are often required in order to correctly deduce an elemental composition for an ion or to otherwise identify an ion, especially for high-mass ions such as those observed by ionization of biochemical compounds as they elute from a chromatographic column. Conventional triple-quadrupole instruments generally provide unit mass resolution. However, resolution to 4-5 decimal places is often required and may be obtained utilizing high performance time-of-flight (TOF) or electrostatic trap mass spectrometers (such as Orbitrap® mass spectrometers). At such resolutions, multiplets resulting from isotope distributions are either partially or fully resolved and often must be accounted for in order to properly assign peaks to ionic species or in the determination of elemental compositions of ionic species.

In principal, elemental composition calculations are simple. If the probability of occurrence of a particular isotope atom of a particular element is given by $p$, then, given a molecule containing $n$ atoms of the element, then the probability, $P(k; n,p)$, that the molecule contains $k$ atoms of the isotope is given by

$$P(k;n,p) = \frac{n!}{k!(n-k)!} p^k (1-p)^{n-k} \qquad \text{Eq.1}$$

Since the isotopic composition of each element in the molecule should be independent of the isotopic composition of every other element in the molecule, then the probability of occurrence of a molecule having a particular isotopic composition considered over all of its elements (and thus a particular mass) may be calculated as a joint probability by taking a product in which the individual factors are determined as in Eq. 1. The results of such model calculations are simulated mass spectra in which all possible peaks of an isotopic distribution are represented at their correct mass values and correct relative intensities.

In actual practice, situations often occur in which little or nothing is known about the molecule whose *m/z* value is being considered. In such a case, a full calculation of all possible elements and all possible isotopes is unwieldy and time-consuming. In the uncommon case where the mass of interest is a major component of the spectrum, a simple inspection of possible isotope patterns ($^{12}C$/$^{13}C$ $^{35}Cl$/$^{37}Cl$ and so on) can detect elements that must be included in the calculation as well as possible abundances of said elements. This approach can even be codified in an automated process that looks for characteristic isotope patterns, and works well for cases where the masses of interest are large and noise is small or absent. However, if the mass of interest is a minor component that is represented by low intensity peaks, then the presence of noise, especially noise of similar magnitude to the signal of interest, changes the situation. A "pattern" might be detected that is nothing but a chance arrangement of chemical or instrumental noise.

The foregoing discussion indicates that there is a need in the art for identifying related peaks - such as peaks related as by an isotopic distribution - in a mass spectrum while discriminating actual peaks from noise interference. One strategy for eliminating noise interference is to search for mass (mass-to-charge ratio) peaks whose elution profiles in time are correlated with one another. One automated calculation method for implementing such a strategy is the technique of Parameterless Peak Detection (PPD) which is described in United States Patent Application publication 2010/0100336 A1 titled "Methods of Automated Spectral Peak Detection and Quantification without User Input" and assigned to the assignee of the present invention. By using PPD, potential chromatographic peaks are rigorously examined and spurious ones eliminated, and multiple quality parameters are available on those peaks which pass, to allow further characterization of these peaks.

Summary

The inventor of the present invention has realized that the technique of Parameterless Peak Detection (PPD) is useful to identify correlations between peaks - and, in particular, isotopic peaks - related to elution of a single ion because all such peaks will exhibit the same chromatographic response and have similar lineshapes in the time domain. Embodiments in accordance with the present teachings may thus address the above-noted needs in the art by providing methods employing a stepwise approach. In one step, peaks are automatically detected by the methods of parameterless peak detection (PPD) and located within each of a plurality of extracted ion chromatograms (XICs) derived from time-based mass spectrometry data obtained during LC/MS analysis. During this process, peak information is retained only for those ions for which chromatographic peaks occur. Further, as peaks are detected, they are subjected to a few quality tests that are unique to XIC data. Since the extracted ion chromatograms should not be complex chromatograms with many overlapping peaks, the first rule is that the area of a peak must be an appreciable fraction of the area remaining in the XIC. Also, while the PPD technique can do an excellent job of extracting peak shapes from large "lumpy" regions, such features are not to be expected in extracted ion chromatograms, and, therefore, an additional test is employed such that each peak intensity must large with respect to an average intensity, which may comprise a local average intensity, a global average intensity or some average calculated on the basis of both local and global values. These constraints are particularly effective in reducing "noise" when employed with XIC data. Accordingly, this step provides a significant data size reduction. The result or output of this step is either a filtered data file written to computer-readable media, or a list of components found in the original data, or both. The automatic peak detection and location techniques do not make *a priori* assumptions about the particular line shape of the chromatographic or spectroscopic peak(s) and may fit any individual peak to either a Gaussian, exponentially modified Gaussian, Gamma distribution or to another form or to a composite form comprising more than one of the above peak forms.

In a subsequent step, the remaining ions are grouped by calculating the cross correlations of relevant parameters pairwise between the various remaining peaks. To perform this calculation, a vector is constructed for each peak, and a correlation coefficient is computed between each vector and every other vector. In some embodiments, each vector may consist of several variables including the peak width, and 9 intensity values obtained from the parametric determination of peak shape. The time points of the intensity values cover the region of the XIC where PPD has determined that a peak exists. The correlation analyses are performed simply on mass spectrometric "full scans" in which broad ranges of masses are monitored simultaneously. This means that this new method of isotope multiplet detection is applicable to any mass spectrometer with chromatographic input and capable of full-scan MS recording.

According to first aspect of the invention, there is provided a method for automatically identifying groups of related peaks generated in a chromatography - mass spectrometry experiment comprises automatically choosing a time window defining a region of interest for mass spectral data generated by the experiment; automatically constructing a plurality of extracted ion chromatograms (XICs) for mass spectral peaks observed within the region of interest; automatically detecting and characterizing chromatogram peaks within each XIC and automatically generating synthetic analytical fit peaks thereof; automatically discarding a subset of the synthetic analytical peaks which do not satisfy noise reduction rules; automatically performing a respective cross-correlation score calculation between each pair of synthetic analytical fit peaks; and automatically identifying groups of correlated peaks in at least one mass spectral scan within the region of interest. In embodiments, the step of automatically identifying groups of correlated peaks in at least one mass spectrum within the region of interest may include automatically identifying at least one group of peaks that corresponds to an isotopic distribution. An automatic identification of a chemical composition of an ionic species may be based upon the automatic identification of the at least one group of peaks that corresponds to the isotopic distribution. In embodiments, the step of automatically identifying groups of correlated peaks in at least one mass spectrum within the region of interest may include automatically identifying a first group of peaks that corresponds to a first isotopic distribution and a second group of peaks that corresponds to a second isotopic distribution, wherein the first and second groups of peaks are associated with elution of a first and a second analyte, respectively, wherein the first and second analytes co-elute.

According to a second aspect of the invention, there is provided an apparatus comprising: a chromatograph for providing a stream of at least partially separated chemical substances; a mass spectrometer fluidically coupled to the chromatograph for generating and analyzing a plurality of types of ions, each type of ion comprising a different mass-to-charge ($m/z$) ratio resulting from ionization of each of the chemical substances; a detector for detecting abundance data for each type of ion; and a programmable electronic processor electrically coupled to the mass spectrometer, the programmable processor comprising instructions operable to cause the programmable processor to: receive the abundance data for each of the types of ions; automatically detect and characterize chromatogram peaks as a function of time for each of a plurality of $m/z$ ratio ranges of the abundance data; automatically generate synthetic analytical fit peaks to the detected chromatogram peaks; automatically discard a subset of the synthetic analytical fit peaks which do not satisfy noise reduction rules; automatically perform a respective cross-correlation score calculation between each pair of synthetic analytical fit peaks; and automatically identify groups of correlated peaks in at least one mass spectrum within the region of interest.

Brief description of the Drawings

The above noted and various other aspects of the present invention will become apparent from the following description which is given by way of example only and with reference to the accompanying drawings, not drawn to scale, in which:

FIG. 1 is a schematic diagram of a system for generating and automatically analyzing chromatography / mass spectrometry spectra in accordance with the present teachings;

FIG. 2 is a schematic illustration of an example of a mass spectrometer system comprising an electrostatic trap mass analyzer such as an Orbitrap® mass analyzer;

FIG. 3 is a perspective view of a three-dimensional graph of chromatography-mass spectrometry data, in which the variables are time, mass (or mass-to-charge ratio, m/z) and ion abundance;

FIGS. 4A-4B provide a flowchart of a method for automatically identifying correlations between ions in accordance with the present teachings;

FIG. 5 is a flowchart of a method for automated spectral peak detection and quantification in accordance with the present teachings;

FIG. 6 is a flowchart of a method for automatically removing baseline features and estimating background noise from spectral data in accordance with the present teachings;

FIG. 7 is a graph of an example of the variation of the calculated area underneath a baseline-corrected spectral curve as a function of the order of polynomial used in fitting the baseline to a polynomial function;

FIG. 8 is an example of a preliminary baseline corrected spectral curve prior to fitting the end regions to exponential functions and an example of the baseline comprising exponential fit functions;

FIG. 9 is a flowchart of a method for automated spectral peak detection and quantification in accordance with the present teachings;

FIGS. 10A-10C are graphical examples of discrimination of peaks of interest from noise peaks in an ion chromatogram;

FIG. 11 a graph of a hypothetical skewed spectral peak depicting a method in accordance with the present teachings for obtaining three points on the spectral peak to be used in an initial estimate of skew and for preliminary peak fitting;

FIG. 12 a graph of a set of gamma distribution functions having different values of shape parameter M, illustrating a fashion by such functions may be used to synthetically fit skewed spectral peaks;

FIG. 13 is a flowchart illustrating a method for choosing between line shapes used for fitting;

FIG. 14A is a flowchart illustrating steps for estimating coordinates of points at a peak maximum and along flanks of the peak at half-height, according to a method of the present teachings;

FIG. 14B is a flowchart illustrating alternative steps for estimating coordinates of points at a peak maximum and along flanks of the peak at half-height, according to a method of the present teachings;

FIG. 15 is a perspective view of a three-dimensional graph of chromatography-mass spectrometry data showing two hypothetical mass spectra of ions and showing hypothetical extracted ion chromatograms (XICs) for several different values of mass-to-charge ratio;

FIG. 16 is a set of plots of several observed line shapes in various extracted ion chromatograms obtained from LC/MS data covering the 1.7-second elution of a single mass chromatographic peak (e.g., a total ion chromatogram peak) of a 500 nM solution of the drug Buspirone;

FIG. 17 is a schematic illustration of two peaks having differing line shapes illustrating a method of calculating a cross-correlation score as a dot product;

FIG. 18A is a full-scan mass spectrum of a chromatographically separated component of a urine solution;

FIG. 18B is a subset of the mass spectral peaks of FIG. 18A identified by performing an automated correlation analysis in accordance with the present teachings;

FIG. 19A is a full-scan mass spectrum of a second chromatographically separated component of the same urine solution used to generate the mass spectrum of FIG. 18A;

FIG. 19B is a subset of the mass spectral peaks of FIG. 19A identified by performing an automated correlation analysis in accordance with the present teachings;

FIG. 20A is a full-scan mass spectrum of a chromatographically separated component of a bile solution; and

FIG. 20B is a subset of the mass spectral peaks of FIG. 20A identified by performing an automated correlation analysis in accordance with the present teachings.

Detailed Description

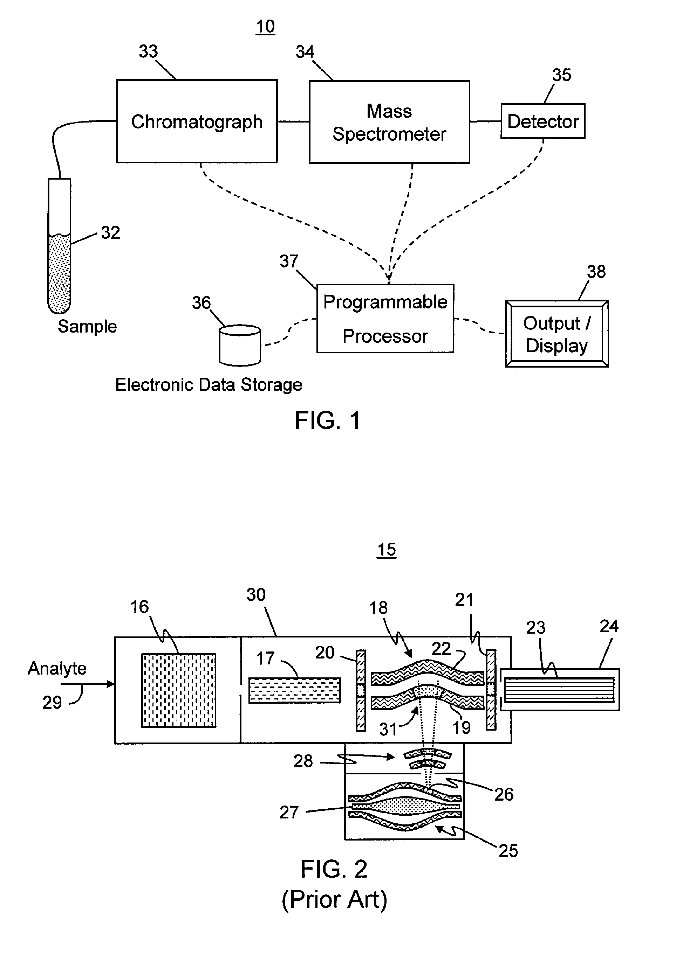

The present invention provides methods and apparatus for correlating ions observed in liquid chromatography / mass spectrometry data. The automated methods and apparatus described herein do not require any user input or intervention. The following description is presented to enable any person skilled in the art to make and use the invention, and is provided in the context of a particular application and its requirements. Various modifications to the described embodiments will be readily apparent to those skilled in the art and the generic principles herein may be applied to other embodiments. Thus, the present invention is not intended to be limited to the embodiments and examples shown but is to be accorded the widest possible scope in accordance with the features and principles shown and described. The particular features and advantages of the invention will become more apparent with reference to the appended FIGS. 1-20, taken in conjunction with the following description.

*Section 1. General Considerations*

FIG. 1 is a schematic diagram of a system for generating and automatically analyzing chromatography / mass spectrometry spectra in accordance with the present teachings. A chromatograph **33,** such as a liquid chromatograph, high-performance liquid chromatograph or ultra high performance liquid chromatograph receives a sample **32** of an analyte mixture and at least partially separates the analyte mixture into individual chemical components, in accordance with well-known chromatographic principles. As a result, the at least partially separated chemical components are transferred to a mass spectrometer **34** at different respective times for mass analysis. As each chemical component is received by the mass spectrometer, it is ionized by an ionization source of the mass spectrometer. The ionization source may produce a plurality of ions comprising differing charges or masses from each chemical component. Thus, a plurality of ions of differing mass-to-charge ratios may be produced for each chemical component, each such component eluting from the chromatograph at its own characteristic time. These various ions are analyzed and detected by the mass spectrometer together with its detector **35** and, as a result, appropriately identified according to their various mass-to-charge ratios.

Still referring to FIG. 1, a programmable processor **37** is electronically coupled to the detector of the mass spectrometer and receives the data produced by the detector during chromatographic / mass spectrometric analysis of the sample(s). The programmable processor may comprise a separate stand-alone computer or may simply comprise a circuit board or any other programmable logic device operated by either firmware or software. Optionally, the programmable processor may also be electronically coupled to the chromatograph and/or the mass spectrometer in order to transmit electronic control signals to one or the other of these instruments so as to control their operation. The nature of such control signals may possibly be determined in response to the data transmitted from the detector to the programmable processor or to the analysis of that data. The programmable processor may also be electronically coupled to a display or other output **38,** for direct output of data or data analysis results to a user, or to electronic data storage **36.**

The programmable processor shown in FIG. 1 is generally operable to: receive a chromatography / mass spectrometry spectrum from the chromatography / mass spectrometry apparatus; generate and evaluate a plurality of extracted ion chromatograms (XICs) from the spectral data; automatically subtract a baseline from each such XIC so as to generate a plurality of baseline-corrected XICs; automatically detect and characterize all spectral peaks occurring above a noise level in each baseline-corrected XIC; perform a cross-correlation calculation between each pair of detected peaks; and report or record information relating to the peaks or to the cross-correlations between the peaks.

An example of a mass spectrometer system **15** comprising an electrostatic trap mass analyzer such as an Orbitrap® mass analyzer **25** is shown in FIG. 2. Analyte material **29** is provided to a pulsed or continuous ion source **16** so as to generate ions. Ion source **16** could be a MALDI source, an electrospray source or any other type of ion source. In addition, multiple ion sources may be used. The illustrated system comprises a curved quadrupole trap **18** (also known as a "C-trap") with a slot **31** in the inner electrode **19.** Ions are transferred from the ion source **16** to the curved quadrupole trap **18** by ion optics assembly **17** (e.g. an RF multipole). Prior to ion injection, ions may be squeezed along the axis of the curved quadrupole trap **18** by raising voltages on end electrodes **20** and **21.** For ion injection into the Orbitrap® mass analyzer **25,** the RF voltage on the curved quadrupole trap **18** may be switched off, as is well known. Pulses are applied to electrodes **19** and **22** and to an electrode of curved ion optics **28** so that the transverse electric field accelerates ions into the curved ion optics **28.** The converging ion beam that results enters the Orbitrap® mass analyzer **25** through injection slot **26.** The ion beam is squeezed towards the axis by an increasing voltage on a central electrode **27.** Due to temporal and spatial focusing at the injection slot **26,** ions start coherent axial oscillations. These oscillations produce image currents that are amplified and processed. Further details of the electrostatic trap apparatus **25** are described in International Application Publication WO 02/078046, US Pat. No. 5,886,346, US Pat. No. 6,872,938. The ion optics assembly **17,** curved quadrupole trap **18** and associated ion optics are enclosed in a housing **30** which is evacuated in operation of the system.

The system **15** (FIG. 2) may further comprise a reaction cell **23,** which may comprise a collision cell (such as an octopole) that is enclosed in a gas tight shroud **24** and that is aligned to the curved quadrupole trap **141.** The reaction cell **23,** when used as a collision cell, may be supplied with an RF voltage of which the DC offset can be varied. A collision gas line (not shown) may be attached and the cell is pressurized with nitrogen (or any) gas.

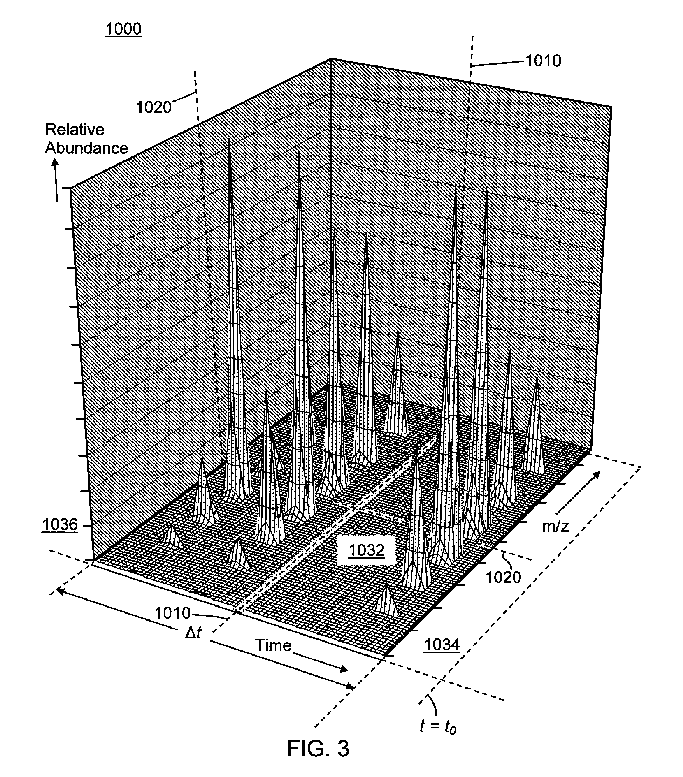

FIG. 3 is a perspective view of a three-dimensional graph of hypothetical LC/MS data. As is common in the representation of such data, the variables time and mass (or mass-to-charge ratio, $m/z$) are depicted on the "floor" of the perspective diagram and the variable representing ion abundance (for instance, detected ion current) is plotted in the "vertical" dimension of the graph. Thus, ion abundance is represented as a function of the other two variables, this function comprising a variably shaped surface above the "floor". Each set of peaks dispersed and in line parallel to the $m/z$ axis represents the various ions produced by the ionization of a single eluting analyte (or, possibly, of fortuitously co-eluting analytes) at a restricted range of time. In a well-designed chromatographic experiment, each analyte of a mixture will elute from the column (thereby to be mass analyzed) within a particular diagnostic time range. Consequently, either a single peak or a line of mass-separated peaks, each such peak representing a particular ion produced by the eluting analyte, is expected at each elution time (or retention time) range.

For clarity, only a very small number of peaks are illustrated in FIG. 3. In practice, data obtained by a chromatography-mass spectrometry experiment may comprise a very large volume of data. A mass spectrometer may generate a complete "scan" over an entire mass range of interest in a matter of tens to hundreds of milliseconds. In this document, the term "scan" is used to refer generally to a mass spectrum, regardless of whether the mass spectrum is obtained by a quadrupole mass spectrometer, a time-of-flight mass spectrometer, a Fourier-transform ion-cyclotron mass spectrometer, an electrostatic trap mass spectrometer (for example, an Orbitrap® mass spectrometer as is sold by Thermo Fisher Scientific corporation of Waltham, Massachusetts USA) or any other type of mass spectrometer. As a result of the speed of many mass spectrometers, up to several hundred complete mass spectra may be generated every second. Further, the various analytes may elute over a time range of several minutes to several tens of minutes, depending on the complexity of the mixture under analysis and the range of retention times represented.

The data depicted in FIG. 3 may comprise an entire stored data file representing results of a prior experiment. Alternatively, the data represent a portion of a larger data set in the process of being acquired by an LC/MS instrument. For instance, the data depicted in FIG. 3 may comprise recently collected data held in temporary computer readable memory, such as a memory buffer, and corresponding to an analysis time window, $\Delta t$, upon which calculations are being formed while, at the same time, newer data is being collected. Such newer, not-yet-analyzed data is represented, in time and $m/z$ space, by region **1034** and the data actually being collected is represented by the line $t=t_0$. Older data, which has already been analyzed by methods of the present teachings and which has possibly been stored to a permanent computer readable medium, is represented by region **1036.** With such manner of operation, methods in accordance with the present teachings may be carried out in near-real-time on an apparatus used to collect the data or using a processor (such as a computer processor) closely linked to the apparatus used to collect the data.

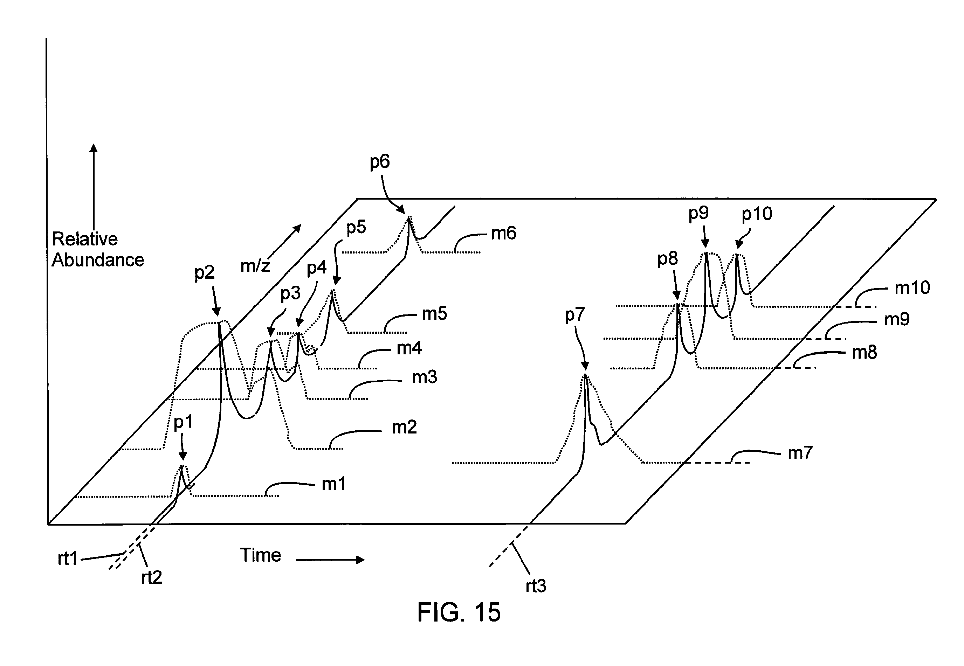

Operationally, data such as that illustrated in FIG. 3 is collected as separate mass spectra (also referred to herein as "scans"), each mass spectrum (scan) corresponding to a particular respective time point. Such mass spectra may be envisioned as residing within planes parallel to the plane indicated by the trace lines **1010** in FIG. 3 or parallel to the lines **rt1**, **rt2** and **rt3** in FIG. 15 (discussed further below). Once at least a portion of data has been collected, such as the data in region **1032** in FIG. 3, then the information in the data portion may be logically re-organized as extracted ion chromatograms (or, at least portions thereof). Each such XIC may be envisioned as a cross section through the data in a plane parallel to the plane indicated by trace lines **1020** in FIG. 3 or parallel to the lines **m1-m10** in FIG. 15. Portions of hypothetical extracted ion chromatograms are shown as dotted lines in FIG. 15. Each XIC represents the elution profile, in time, of ions of a particular mass-to-charge range. The XIC representation of the data is useful for understanding the methods of the present teachings.

Several schematic hypothetical XIC profiles are shown using dotted lines in FIGS. 15. Also, portions of mass scans (i.e., mass spectra) are indicated by solid lines at times indicated by **rt1, rt2** and **rt3.** These profiles and scans include several example peaks. These include peak **p1** at coordinates ($rt2$, $m1$), peak **p2** at coordinates ($rt1$, $m2$), peak **p3** at coordinates ($rt1$, $m3$), peak **p4** at coordinates ($rt1$, $m4$), peak **p5** at coordinates ($rt1$, $m5$), peak **p6** at coordinates ($rt1$, $m6$), peak **p7** at coordinates ($rt3$, $m7$), peak **p8** at coordinates ($rt3$, $m8$), peak **p9** at coordinates ($rt3$, $m9$) and peak **p10** at coordinates ($rt3$, $m10$).

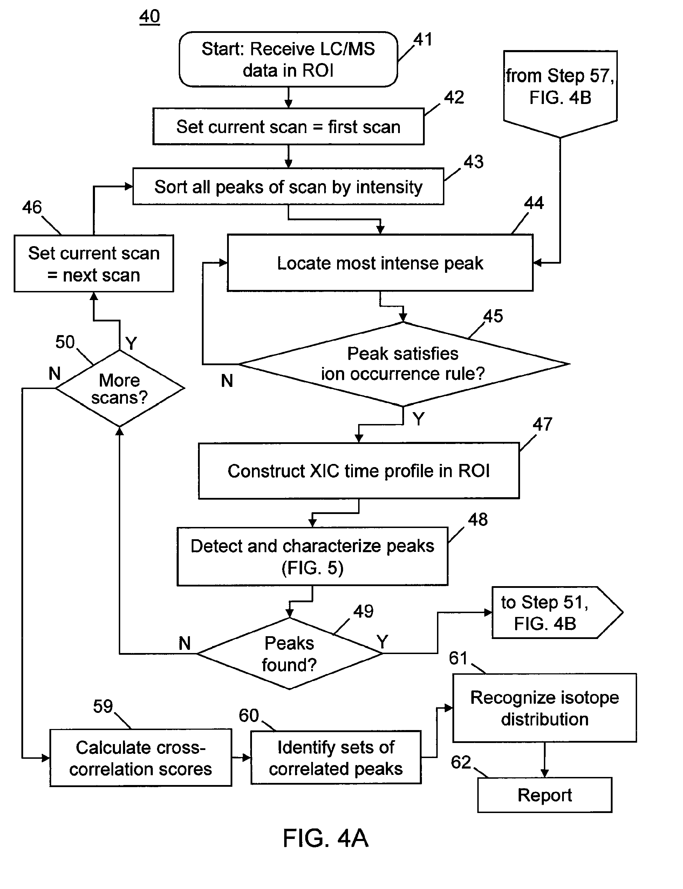

1.1. High-Level Methods. FIGS. 4A-4B present a flowchart of a method **40** for generating automated correlations between the various mass spectral peaks of a related set of peaks in accordance with the present teachings. In the initial step, step **41** (FIG. 4A), mass spectral data is generated by and received from a chromatograph-mass spectrometer apparatus. The LC/MS data comprises ion abundance (or relative abundance) information as a function of time and $m/z$.

The calculations of method **40** are performed on a chosen time window of the data set on scans of a certain type. Scans are restricted to a certain scan type since different types of scans may be interleaved with one another. For instance a scan of precursor ions may be immediately followed by a scan of product ions, wherein the product ions are generated by fragmentation or other reaction of one or several of the precursor ions. The scan of fragment ions may then be followed by another scan of precursor ions and this sequence may be repeated such that precursor scans and product scans alternate with one another.

The chosen time-window corresponds to a current region of interest (ROI) of recently collected data, such as region **1032** of FIG. 3. In embodiments, this window is 1.4 minutes wide, but other window widths will work equally well as long as the window width is greater than the expected peak width. Good results have been obtained by the inventor using window widths of 0.5 minutes and 0.2 minutes. These time windows represent a small portion of a typical chromatographic experiment which may run for several tens of minutes to on the order of an hour. For data dependent instrument control, a much smaller time window would probably be used. Such data dependent instrument control functions may be performed in automated fashion, wherein the results obtained by the methods herein are used to automatically control operation of the instrument at a subsequent time during the same experiment from which the data were collected. For instance, based on an ion identification provided by the results of the algorithms, a voltage may be automatically adjusted in an ion source or a collision energy (that is applied to ions in order to cause fragmentation) may be adjusted with regard to collision cell operation. Such automatic instrument adjustments may be performed, for instance, so as to optimize the type or number of ions or ion fragments produced by fragmentation of the identified ion.

At a high- or most-general level, any algorithm that systematically examines the data of the region of interest in the time window, searching for peaks to be tested by subsequent cross-correlation calculation, may be employed. For example, an algorithm may march through the data, scan-by-scan. In the present example, the window width is only 0.7 minutes wide at time zero since there is no data before time=0. As scans of higher time are examined, the window increases until the scan at time 0.7 minutes uses a window of the specified 1.4 minutes.

In step **42** of the present example (FIG. 4A), the scan to be examined (the current scan) is set to be the initial scan of a certain type (for instance, full-MS scan type, precursor ion scan type, etc.) within the ROI. This is an initialization step for a loop in which scans of the chosen type are sequentially examined. In step **43,** the peaks of the current scan are sorted by intensity and the ions are examined one by one, starting with the most intense (step **44).** In general, all ions are examined, but for very rapid work or strong signals, a threshold may be applied and only ions with intensities above threshold examined. In the present example, step **59** (described in greater detail later in this document) is performed when all peaks within the ROI have been identified. In Step **45** of this example, the occurrence of a feature - a candidate ion-related peak - in the spectrum is noted, and its history or time-profile is compared to a rule for ions to be considered as forming a peak. A preferred rule that is used is that the spectral feature must occur in three successive scans (scans of the same type), but any rule based on ion appearance and scan number may be used. For example, a rule that the ion must appear in 3 of 5 successive scans of the same type might alternatively be chosen. (Ions are considered identical if they agree within the mass tolerance, and as an ion history is accumulated, any new occurrence is compared to the average value of the previous instances, not simply the previous instance.)

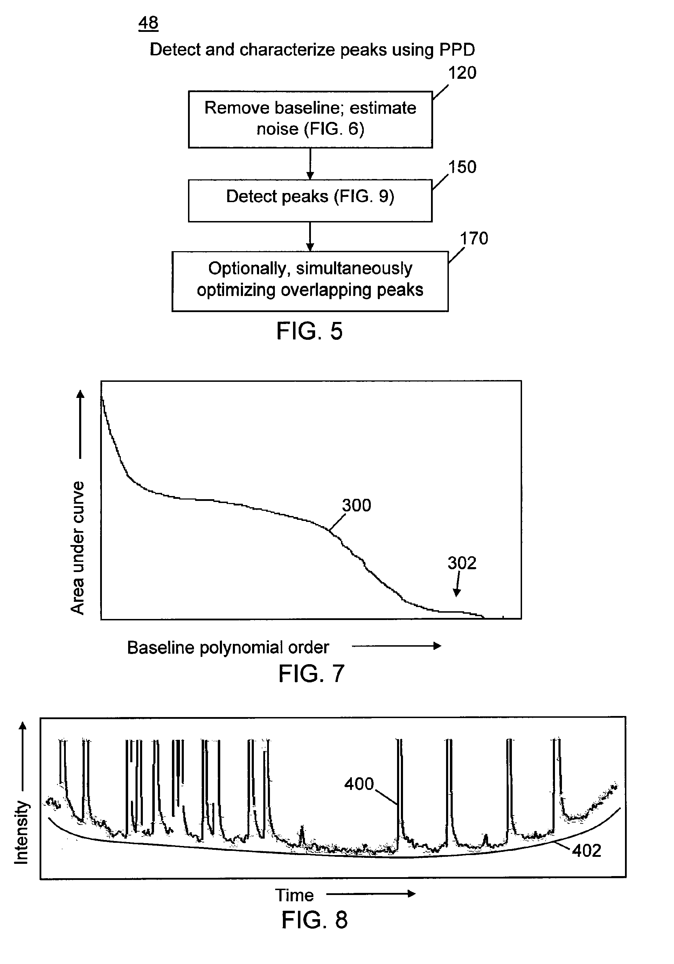

If, in step **45,** the peak under consideration does not satisfy the ion occurrence rule, then the method returns to step **44** to examine the next most intense peak (corresponding to another ion) in the current scan. If the ion occurrence rule (as determined in step **45)** is satisfied, then an extracted ion chromatogram corresponding to the *m/z* range of the ion peak under consideration is constructed in step **47.** It is to be noted that the terms "mass" and "mass-to-charge" ratio, as used here, actually represent a small finite range of mass-to-charge ratios. The width or "window" of the mass-to-charge range is the stated precision of the mass spectrometer instrument. The technique of Parameterless Peak Detection (PPD, see FIG. 5 and discussion thereof as well as United States Patent Application publication 2010/0100336 A1) then attempts to find peaks in an extracted ion chromatogram (XIC) corresponding to this 1.4 minute time window in step **48.** Once this particular mass has been tested for peaks in the XIC, it is not tested again until the center of the time window has increased by the window size. (So, for example, if an ion is tested for peaks when the time window is 2-3.4, it will not be tested again until the window is 3.4-4.8.)

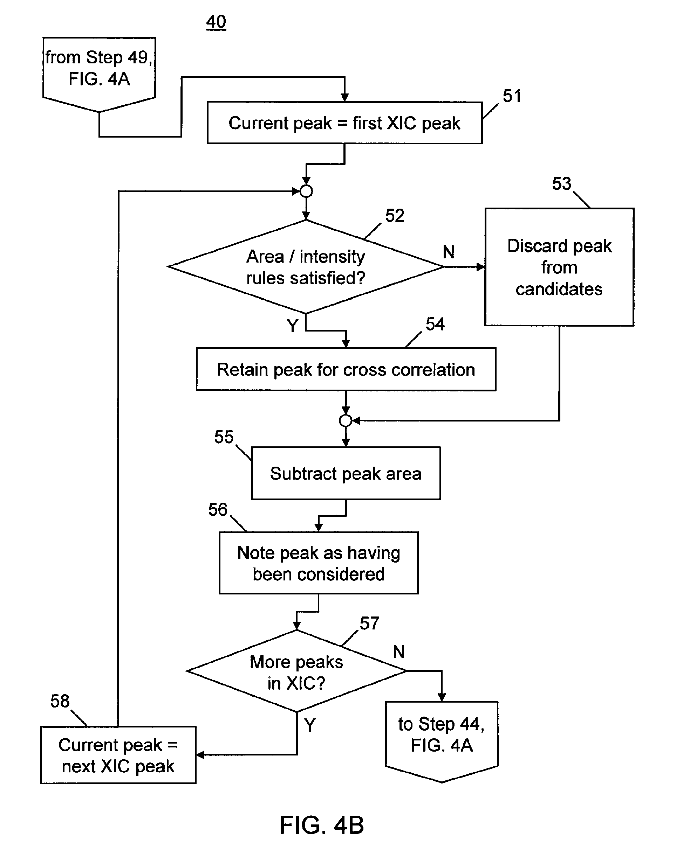

Subsequent steps of the method **40** are performed using the analytical functions provided by the synthetic fitted peaks generated by PPD (or calculated peak parameters) instead of using the original data. If, in the decision step **49,** no peaks are found by PPD for the mass under consideration, then, if there are remaining unexamined scans of the same type (step **50),** the method returns back to step **46** and then step **43.** The decision step **49** as to whether any peaks occur in the XIC may include a statistical test wherein an intensity of candidate peaks are compared to an average value of a running list of the intensities of recently found peaks for ions of different mass-to-charge ratios. If peaks are found to occur in the XIC, then the method continues to step **51** (FIG. 4B) in which the first of possibly several peaks in the XIC is set for initial consideration. In the next step **52,** for each peak found by PPD, additional rules of large relative area and high relative intensity (described in further detail in the next paragraph) are applied. Peaks that fail these tests are discarded (step **53),** whereas those that pass are accepted and retained (step **54)** for further processing by cross-correlation score calculations (such correlation scores are calculated in step **59).** Regardless of whether or not a peak is accepted, after each peak is considered, the peak area of the peak is subtracted (step **55)** from the total area used in the relative area criterion in subsequent iterations of step **52.** Also (step **56)** the peak is added to a list of peaks within the ROI that have been examined, to prevent possible duplicate consideration of a single peak.

The step **52** of the method **40** is now discussed in more detail. In step **52,** the area, $A_j$, of the peak currently under consideration (the jth peak) is noted. Also, the total area ($\Sigma A$) under the curve of the fitted chromatogram and the average peak height ($I_{ave}$) of any remaining peaks in the fitted chromatogram are calculated. The area $\Sigma A$ is the area of the data remaining after any previous peaks have been detected and removed. The step **52** compares the area, $A_j$, of the most recently found peak to the total area ($\Sigma A$) where the summation may be taken over the entire XIC, over a local area in the region of the found peak or according to a criterion based on both global and local areas. Also, this step compares the peak maximum intensity, $I_j$, of the most recently found peak is compared to $I_{ave}$, where the average may be calculated over the entire XIC, over a local region in the vicinity of the found peak or according to a criterion based on both global and local regions. If it is found either that $(A_j / \Sigma A) < \omega$ or that $(I_j / I_{ave}) < \rho$, where $\omega$ and $\rho$ are pre-determined constants, then the execution of the method **40** branches to step **53** in which the peak is removed from a list of peaks to be considered in - and is thus eliminated from consideration in - the subsequent cross-correlation score calculation step.

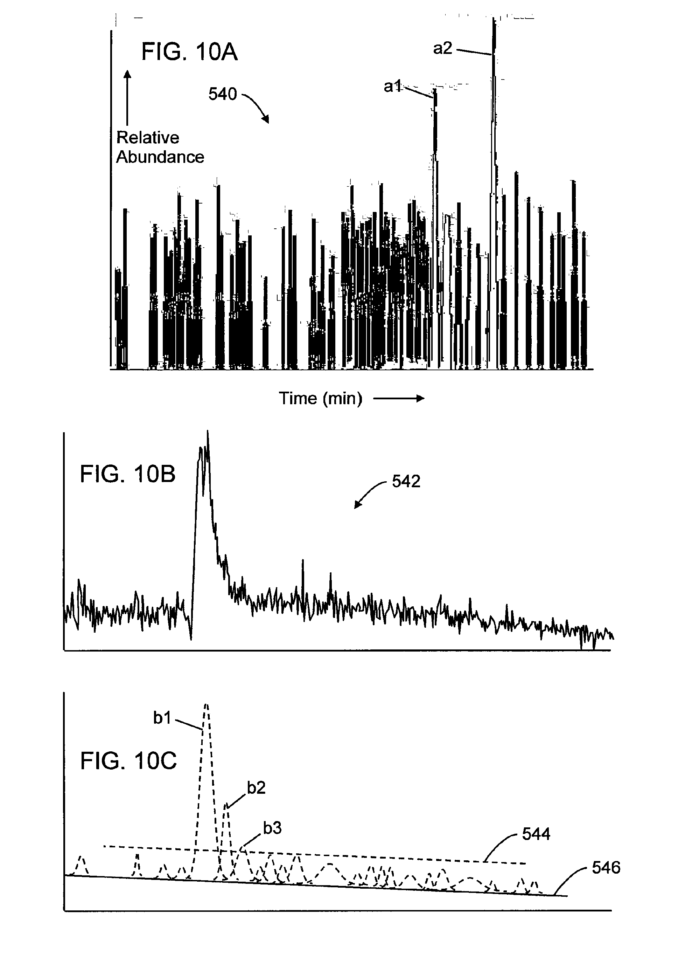

The removal of certain peaks in this fashion renders the fitted peak set consistent with the expectations that, within an XIC, each actual peak of interest should comprise a significant peak area, relative to the total peak area and should comprise a vertex intensity that is significantly greater than an average intensity. As noted above, the average intensity calculated according to a criterion based on both global and local values, as noted above. FIGS. 10A-10C schematically illustrate this concept. For instance, after peak discrimination in step **52** (FIG. 4B), fitted peaks corresponding to data peaks **a1** and **a2** in of the XIC **540** in FIG. 10A may, in some embodiments, not be retained in the list of peaks to be tested by cross correlation as a result of their relatively smaller peak areas in relation to the total area above the baseline. In various embodiments, the retention of peaks may be determined based on statistical considerations - such as correlation statistics between different data files - or possibly some other criteria related to relative peak areas. Numerous fitted peaks in FIG. 10C, which represent a fit to the XIC **542** of FIG. 10B, are eliminated by a different criterion. For example, all fitted peaks in FIG. 10C that do not extend above line **544** may be eliminated because their peak heights do not meet a peak height criterion, even though the areas of several of them are not insignificant. In the illustrated example, line **546** is a baseline and line **544** is a line offset from the baseline such that the vertical distance between the two lines represents a minimum peak height for acceptance. Thus, in this case, only peaks **b1, b2** and **b3** are retained. In various embodiments, the retention of peaks may be determined based on statistical considerations or some other criteria related to relative peak heights.

Returning to the discussion of the method **40** (FIG. 4B), it may be noted that if the decision step **57** determines that more peaks exist in the XIC under consideration, then the method branches to step **58** in which the next peak is set for consideration and then back to step **52**. The decision step **57** as to whether another XIC peak exists may include a statistical test wherein an intensity of a candidate peak is compared to an average value of a running list of the intensities of recently found peaks in the XIC or found for different mass-to-charge ratios. If it is determined that no additional peaks remain the XIC, then execution goes back to step **44** (FIG. 4A) so as to continue examining additional peaks (if any) in the current scan. The above-described sequence continues until all peaks in all scans of the chosen type have been examined and, consequently, all peaks to be used for matching have been identified. Subsequently, in Step **59,** the cross correlation for each retained XIC peak is calculated with respect to every other mass that formed an XIC peak in the region of interest. The details of the calculations are presented in a subsequent section herein. In step **60,** sets or clusters of correlated peaks within the region of interest are identified, based on the cross-correlation scores calculated in step **59**. Although not all peaks that are correlated are necessarily related to one another as components of an isotopic distribution of peaks, certain clusters of mass spectral peaks within a set of correlated peaks may nonetheless be either positively identified as or be noted as candidates for components of an isotopic distribution (step **61**). An isotopic distribution relating to a particular element may, in many cases, be automatically recognized by simple pattern recognition that considers both the spacing and relative intensities of peaks of a group or cluster of peaks. Such patterns may be coded into the recognition routine for automatic recognition.

Finally, in step **62,** the results are reported to a user (or stored for later use). The results may include information regarding detected peaks of an identified isotopic distribution of peaks or of a candidate isotopic distribution. The candidate distributions reported in this fashion may be further analyzed by an expert user. The reporting step **62** may possibly include the reporting of other information. The reporting may be performed in numerous alternative ways-for instance via a visual display terminal, a paper printout, or, indirectly, by outputting the parameter information to a database on a storage medium for later retrieval by a user. The reporting step may include reporting either textual or graphical information, or both. Reported peak parameters may be either those parameters calculated during the peak detection step or quantities calculated from those parameters and may include, for each of one or more peaks, location of peak centroid, location of point of maximum intensity, peak half-width, peak skew, peak maximum intensity, area under the peak, a list of correlated peaks which may match an isotopic distribution pattern, etc. Other parameters related to signal to noise ratio, statistical confidence in the results, goodness of fit, etc. may also be reported in step **62.** Such information may include chemical identification of one or more analytes (e.g., ions, molecules or chemical compounds), purity of analytes, identification of contaminating compounds, ions or molecules or, even, a simple notification that an analyte is (or is not) present in a sample at detectable levels.

*Section 2. Parameterless Peak Detection in One Independent Variable*

The method **40** diagrammed in FIGS. 4A-4B provides a high-level overview of generating automated correlations between ion mass peaks. However, to fully appreciate the features of the invention, it is necessary to provide a significantly more detailed discussion of the step **48** of method **40** as well as additional procedures subsumed therein. The step **48** includes detecting and locating peaks in various extracted-ion-chromatogram (XIC) representations of the mass spectral data and may itself be regarded as a particular method, which is shown in flowchart form in FIG. 5. Since each XIC includes only the single independent variable of time (e.g., Retention Time), this section is thus directed to detection of peaks in data that includes only one independent variable. Much of the discussion in the present section is adapted from the discussion in the aforementioned United States Patent Application publication 2010/0100336 A1.

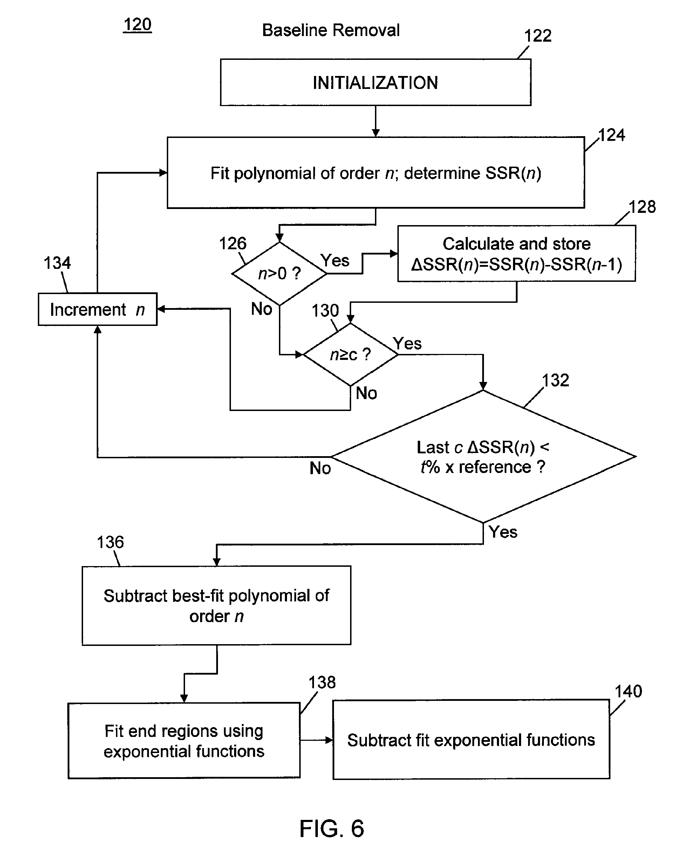

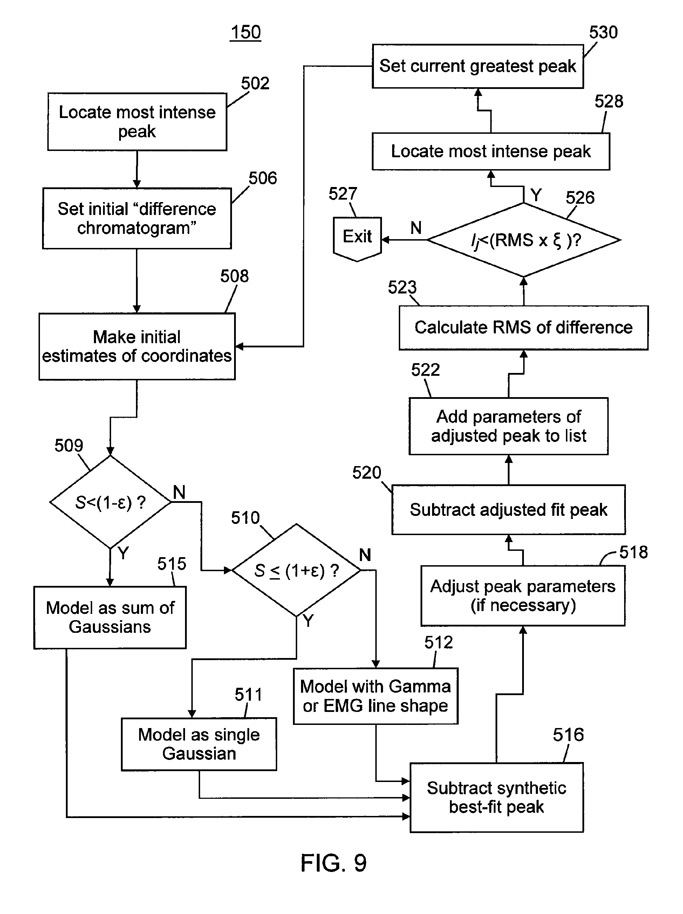

The various sub-procedures or sub-methods in the method **48** may be grouped into three basic stages of data processing, each stage possibly comprising several steps as illustrated in FIG. 5. The first step, step **120,** of the method **48** is a preprocessing stage in which baseline features may be removed from the received chromatogram and in which a level of random "noise" of the chromatogram may be estimated, this step being described in greater detail in subsequent FIG. 6. The next step **150,** which is described in greater detail in subsequent FIG. 9, is the generation of an initial estimate of the parameters of synthetic peaks, each of which models a positive spectral feature of the baseline corrected chromatogram. Such parameters may relate, for instance, to peak center, width, skew and area of modeled peaks, either in preliminary or intermediate form. The subsequent optional step **170** includes refinement of fit parameters of synthetic peaks determined in the preceding step **150** in order to improve the fit of the peaks, taken as a set, to the baseline corrected chromatogram. The need for such refinement may depend on the degree of complexity or accuracy employed in the execution of modeling in step **150.**

The term "model" and its derivatives, as used herein, may refer to either statistically finding a best fit synthetic peak or, alternatively, to calculating a synthetic peak that exactly passes through a limited number of given points. The term "fit" and its derivatives refer to statistical fitting so as to find a best-fit (possibly within certain restrictions) synthetic peak such as is commonly done by least squares analysis. Note that the method of least squares (minimizing the chi-squared metric) is the maximum likelihood solution for additive white Gaussian noise. More detailed discussion of individual method steps and alternative methods is provided in the following discussion and associated figures.

2.1. Baseline Detection. A feature of a first stage of the method **48** (FIG. 5) takes note of the concept that (disregarding, for the moment, any chemical or electronic noise) a spectroscopic signal generally consists of signal plus baseline. If one can subtract the baseline correctly, everything that remains must be signal, and should be fitted to some sort of data peak. Thus, the first step **120** comprises determining a correct baseline and removing it from the signal. Sub-steps may include applying a polynomial curve as the baseline curve, and measuring the residual (the difference between the chromatographic data and the computed baseline) as a function of polynomial order. For instance, FIG. 6 illustrates a flowchart of a method **120** for automatically removing baseline features from spectral data in accordance with some embodiments of the invention. The method **120** illustrated in FIG. 6 repeatedly fits a polynomial function to the baseline, subtracts the best fit polynomial function from the chromatogram so as to provide a current baseline-corrected chromatogram, evaluates the quality of the fit, as measured by a sum of squared residuals (SSR), and proceeds until SSR changes, from iteration to iteration, by less than some pre-defined percentage of its original value for a pre-defined number of iterations.

FIG. 7 is an exemplary graph **300** of the variation of the calculated area underneath a baseline-corrected spectral curve as a function of increasing order of the polynomial used in fitting the baseline. FIG. 7 shows that the area initially decreases rapidly as the order of the best fit polynomial increases. This function will go from some positive value at order zero, to a value of zero at some high polynomial order. However, as may be observed from FIG. 7, after most of the baseline curvature has been fit, the area function attains a plateau region **302** for which the change in the function between polynomial orders is some relatively small amount (for instance 5% of its initial value). At this point, the polynomial-fitting portion of the baseline determination routine may be terminated.

To locate the plateau region **302** as indicated in FIG. 7, methods according to the present teachings may repeatedly compute the sum of squared residuals (SSR) for sequential values of polynomial order, each time computing the difference of the SSR ($\Delta$SSR) determined between consecutive polynomial orders. This process is continued until a region is found in which the change ($\Delta$SSR) is less than the pre-defined percentage (for instance, 5%) of a certain reference value determined from the chromatogram for a certain number $c$ (for instance, four) of sequential iterations. The reference value may comprise, for instance, the maximum intensity of the original raw chromatogram or extracted ion chromatogram. Alternatively, the reference value may comprise the sum of squared values ($SSV_0$) of the chromatogram or some other quantity calculated from the spectral values.

Once it has been found that $\Delta$SSR is less than the pre-defined percentage of the reference value for $c$ iterations, then one of the most recent polynomial orders (for instance, the lowest order of the previous four) is chosen as the correct polynomial order. The subtraction of the polynomial with the chosen order yields a preliminary baseline corrected chromatogram, which may perhaps be subsequently finalized by subtracting exponential functions that are fit to the end regions. Although the above-discussion regarding baseline removal is directed to the general case, it should be noted that the mere construction of an XIC representation eliminates signal from most interfering ions. Thus, the magnitudes of baseline offset and baseline curvature are generally minimal for such data representations.

Returning, now, to the discussion of method **120** shown in FIG, 6, it is noted that the first step **122** comprises a loop initialization step of setting the order, $n$, of the baseline fitting polynomial to an initial value of zero and determining a reference value to be used, in a later step **132,** for determining when the fitting polynomial provides an adequate fit to the baseline. The reference value may simply be the maximum intensity of the extracted ion chromatogram. Alternatively, the reference value may be some other measure determined from the chromatogram, such as the sum of the squared values (SSV) of the chromatogram.

From step **122,** the method **120** proceeds to a step **124** which is the first step in a loop. The step **124** comprises fitting a polynomial of the current order (that is, determining the best fit polynomial of the current order) to the chromatogram by the well-known technique of minimization of a sum of squared residuals (SSR). The SSR as a function of $n$, SSR($n$) is stored at each iteration for comparison with the results of other iterations.

From step **124,** the method **120** proceeds to a decision step **126** in which, if the current polynomial order $n$ is greater than zero, then execution of the method is directed to step **128** in order to calculate and store the difference of SSR, $\Delta$SSR($n$), relative to its value in the iteration just prior. In other words, $\Delta$SSR($n$)=SSR($n$)-SSR($n$-1). The value of $\Delta$SSR($n$) may be taken a measure of the improvement in baseline fit as the order of the baseline fitting polynomial is incremented to $n$.

The iterative loop defined by all steps from step **124** through step **132,** inclusive, proceeds until SSR changes, from iteration to iteration, by less than some pre-defined percentage, $t\%$, of the reference value for a pre-defined integer number, $c$, of consecutive iterations. Thus, the number of completed iterations, integer $n$, is compared to $c$ in step **130.** If $n \geq c$, then the method branches to step **132,** in which the last $c$ values of $\Delta SSR(n)$ are compared to the reference value. However, in the alternative situation ($n < c$), there are necessarily fewer than $c$ recorded values of $\Delta SSR(n)$, and step **132** is bypassed, with execution being directed to step **134,** in which the integer $n$ is incremented by one.

The sequence of steps from step **124** up to step **132** (going through step **128,** as appropriate) is repeated until it is determined, in step **132,** that the there have been $c$ consecutive iterations in which the SSR value has changed by less than $t\%$ of the reference value. At this point, the polynomial portion of baseline correction is completed and the method branches to step **136,** in which the final polynomial order is set and a polynomial of such order is subtracted from the extracted ion chromatogram to yield a preliminary baseline-corrected chromatogram.

The polynomial baseline correction is referred to as "preliminary" since edge effects may cause the polynomial baseline fit to be inadequate at the ends of the data, even though the central region of the data may be well fit. FIG. 8 shows an example of such a preliminary baseline corrected chromatogram **400**. The residual baseline curvature within the end regions (for instance, the leftmost and rightmost 20% of the chromatogram) of the chromatogram **400** are well fit by a sum of exponential functions (one for each end region), the sum of which is shown in FIG. 8 as curve **402**. Either a normal or an inverted (negated) exponential function may be employed, depending on whether the data deviates from zero in the positive or negative direction. This correction may be attempted at one or both ends of the chromatogram. Thus, the method **120** proceeds to step **138** which comprises least squares fitting of the end region baselines to exponential functions, and then to step **140** which comprises subtraction of these functions from the preliminary baseline-corrected chromatogram to yield the final baseline corrected chromatogram. These steps yield a final baseline-corrected chromatogram.

2.2. Peak Detection. At this point, after the application of the steps outlined above, the baseline is fully removed from the data and the features that remain within the chromatogram above the noise level may be assumed to be analyte signals. The methods described in FIG. 9 locate the most intense region of the data, fit it to one of several peak shapes, remove that theoretical peak shape from the experimental data, and then continue to repeat this process until there are no remaining data peaks with a signal-to-noise ratio (SNR) greater than some pre-determined value, $s$, greater than or equal to unity. The steps of this process are illustrated in detail in FIG. 9 as method **150** and also shown in FIG. 5 as step **150**. The pre-defined value, $s$, may be chosen so as to limit the number of false positive peaks. For instance, if the RMS level of Rayleigh-distributed noise is sigma, then a peak detection threshold, $s$, of 3 sigma leads to a false detection rate of about 1%.

The method **150,** as shown in FIG. 9 is an iterative process comprising initialization steps **502** and **506** and loop steps **508-530** (including loop exit decision step **526)** and termination step 527. A new respective peak is located and modeled during each iteration of the loop defined by the sequence of steps **508-530.**

The first step **502** of method **150** comprises locating the most intense peak in the final baseline-corrected chromatogram (of an extracted ion chromatogram) and setting a program variable, current greatest peak, to the peak so located. It is to be kept in mind that, as used in this discussion, the acts of locating a peak or chromatogram, setting or defining a peak or chromatogram, performing algebraic operations on a peak or chromatogram, etc. implicitly involve either point-wise operations on sets of data points or involve operations on functional representations of sets of data points. Thus, for instance, the operation of locating the most intense peak in step **502** involves locating all points in the vicinity of the most intense point that are above a presumed noise level, under the proviso that the total number of points defining a peak must be greater than or equal to four. Also, the operation of "setting" a program variable, current greatest peak, comprises storing the data of the most intense peak as an array of data points.

From step **502,** the method **150** proceeds to second initialization step **506** in which another program variable, "difference chromatogram" is set to be equal to the final baseline-corrected chromatogram (see step **140** of method **120,** FIG. 6). The difference chromatogram is a program variable that is updated during each iteration of the loop steps in method **150** so as to keep track of the chromatogram resulting from subtraction of all prior-fitted peaks from the final baseline-corrected chromatogram. As discussed later in this document, the difference chromatogram is used to determine when the loop is exited under the assumption that, once all peaks have been located and modeled, the difference chromatogram will consist only of "noise".

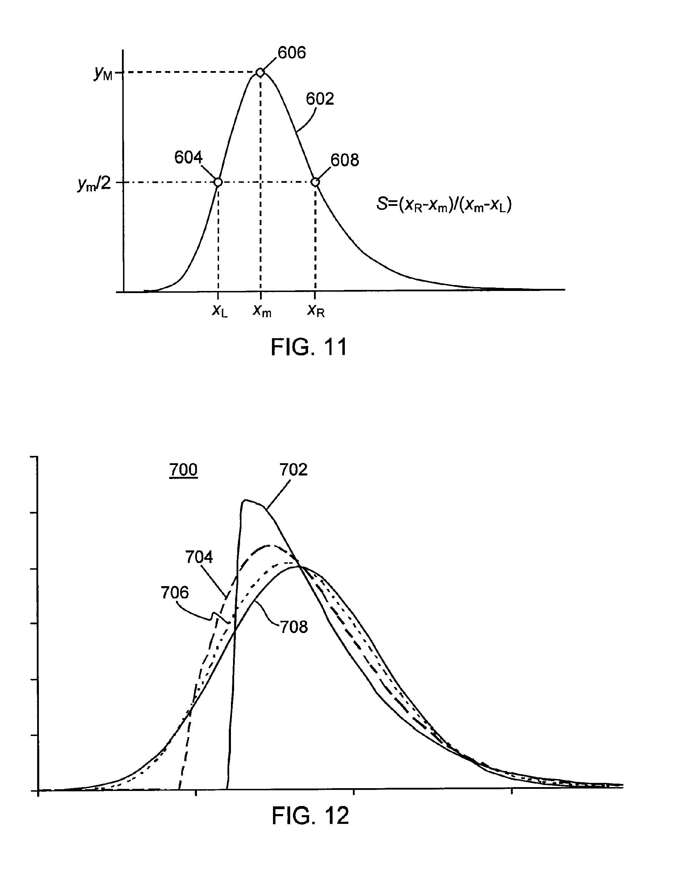

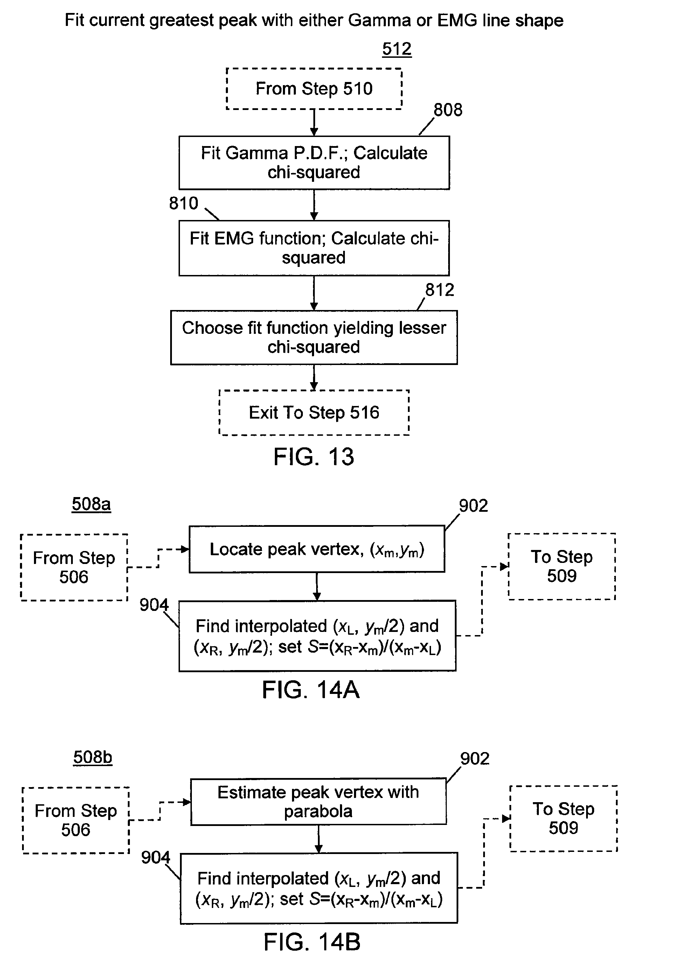

Subsequently, the method **150** enters a loop at step **508,** in which initial estimates are made of the coordinates of the peak maximum point and of the left and right half-height points for the current greatest peak and in which peak skew, $S$ is calculated. The method of estimating these co-ordinates is schematically illustrated in FIG. 11 and is discussed in greater detail later with respect to FIGS. 14A-14B. Letting curve **602** of FIG. 11 represent the current greatest peak, then the co-ordinates of the peak maximum point **606,** left half-height point **606** and right half-height point **608** are, respectively, $(x_m, y_m)$, $(x_L, y_m/2)$ and $(x_R, y_m/2)$. The peak skew, $S$, is then defined as: $S = (x_R - x_m)/(x_m - x_L)$.

In steps **509** and **510,** the peak skew, $S$, may be used to determine a particular form (or shape) of synthetic curve (in particular, a distribution function) that will be subsequently used to model the current greatest peak. Thus, in step **509,** if S < (1-ε), where ε is some pre-defined positive number, such as, for instance, ε =0.05, then the method **150** branches to step **515** in which the current greatest peak is modeled as a sum of two or more Gaussian distribution functions (in other words, two Gaussian lines). Otherwise, in step **510,** if $S \leq (1+\varepsilon)$, then the method **150** branches to step **511** in which a (single) Gaussian distribution function is used as the model peak form with regard to the current greatest peak. Otherwise, the method **150** branches to step **512,** in which either a gamma distribution function or an exponentially modified Gaussian (EMG) or some other form of distribution function is used as the model peak form. Alternatively, the current greatest peak could be modeled as a sum of two or more Gaussian distribution functions in step **512.** A non-linear optimization method such as the Marquardt-Levenberg Algorithm (MLA) or, alternatively, the Newton-Raphson algorithm may be used to determine the best fit using any particular line shape. After either step **511,** step **512** or step **515,** the synthetic peak resulting from the modeling of the current greatest peak is removed from the chromatogram data (that is, subtracted from the current version of the "difference chromatogram") so as to yield a "trial difference chromatogram" in step **516.** Additional details of the gamma and EMG distribution functions and a method of choosing between them are discussed in greater detail, partially with reference to FIG. 13, later in this document.

Occasionally, the synthetic curve representing the statistical overall best-fit to a given spectral peak will lie above the actual peak data within certain regions of the peak. Subtraction of the synthetic best fit curve from the data will then necessarily introduce a "negative" peak artifact into the difference chromatogram at those regions. Such artifacts result purely from the statistical nature of the fitting process and, once introduced into the difference chromatogram, can never be subtracted by removing further positive peaks. However, physical constraints generally require that all peaks should be positive features. Therefore, an optional adjustment step is provided as step **518** in which the synthetic peak parameters are adjusted so as to minimize or eliminate such artifacts.

In step **518** (FIG. 9), the solution space may be explored for other fitted peaks that have comparable squared differences but result in residual positive data. A solution of this type is selected over a solution that gives negative residual data. Specifically, the solution space may be incrementally walked so as to systematically adjust and constrain the width of the synthetic peak at each of a set of values between 50% and 150% of the width determined in the original unconstrained least squares fit. After each such incremental change in width, the width is constrained at the new value and a new least squared fit is executed under the width constraint. The positive residual (the average difference between the current difference chromatogram and the synthetic peak function) and chi-squared are calculated and temporarily stored during or after each such constrained fit. As long as chi-squared doesn't grow beyond a certain multiple of its initial value, for instance 3-times its initial value, the search continues until the positive residual decreases to below a certain limit, or until the limit of peak width variation is reached. This procedure results in an adjusted synthetic fit peak which, in step **520,** is subtracted from the prior version of the difference chromatogram so as to yield a new version of the difference chromatogram (essentially, with the peak removed). In step **522,** information about the most recently adjusted synthetic peak, such as parameters related to peak form, center, width, shape, skew, height and/or area are stored.

In step **523,** the root-of-the-mean squared values (root-mean-square or RMS) of the difference chromatogram is calculated. The ratio of this RMS value to the intensity of the most recently synthesized peak may be taken as a measure of the signal-to-noise (SNR) ratio of any possibly remaining peaks. As peaks continue to be removed (that is, as synthetic fit peaks are subtracted in each iteration of the loop), the RMS value of the difference chromatogram approaches the RMS value of the noise.

Step **526** is entered from step **523.** In step **526,** as each tentative peak is found, its maximum intensity, *I*, is compared to the current RMS value, and if *I* < (RMS x ξ) where ξ is a certain pre-defined noise threshold value, greater than or equal to unity, then further peak detection is terminated. Thus, the loop termination decision step **526** utilizes such a comparison to determine if any peaks of significant intensity remain distinguishable above the system noise. If there are no remaining significant peaks present in the difference chromatogram, then the method **150** branches to the final termination step **527.** However, if data peaks are still present in the residual chromatogram, the calculated RMS value will be larger than is appropriate for random noise and at least one more peak must be fitted and removed from the residual chromatogram. In this situation, the method **150** branches to step **528** in which the most intense peak in the current difference chromatogram is located and then to step **530** in which the program variable, current greatest peak, is set to the most intense peak located in step **528.** The method then loops back to step **508,** as indicated in FIG. 9.

Now that the overall set of steps in the method **150** have been described, the process that is used to model individual spectral features is now discussed in greater detail. Traditional spectral peak fitting routines generally model spectral features using either a Gaussian or Lorentzian forms (commonly referred to as peak shapes or line shapes) and tend to either use one preferred line shape throughout the fitting procedure or to query a user as to which line shape to use. Although any arbitrary peak shape can be modeled with a sum of Gaussians (perhaps requiring some Gaussians with negative intensities), the inventors have observed that commonly occurring natural peak shapes (especially in chromatographic spectral data) include Gaussians or even Gamma-distribution-like functions with tailing or leading edges. Therefore, methods in accordance with the present teachings may employ a library of peak shapes containing at least four curves (and possibly others) to model observed peaks: a Gaussian for peaks that are nearly symmetric; a sum of two Gaussians for peaks that have a leading edge (negative skewness); and either an exponentially modified Gaussian or a Gamma distribution function for peaks that have a tailing edge (positive skewness).

The modeling of spectral peaks with Gaussian line shapes is well known and will not be described in great detail here. Methods in accordance with the present teachings may use a Gaussian functional form that utilizes exactly three parameters for its complete description, these parameters usually being taken as area $A$, mean $\mu$ and variance $\sigma^2$ in the defining equation:

$$I\left(x; A, \mu, \sigma^2\right) = \frac{A}{\sigma\sqrt{2\pi}} \exp\left(-\frac{(x-\mu)^2}{2\sigma^2}\right). \qquad \text{Eq. 2}$$

in which $x$ is the variable of spectral dispersion (generally the independent variable or abscissa of an experiment or spectral plot) such as wavelength, frequency, or time and $I$ is the spectral ordinate or measured or dependent variable, possibly dimensionless, such as intensity, counts, absorbance, detector current, voltage, etc. Note that a normalized Gaussian distribution (having a cumulative area of unity and only two parameters-mean and variance) would model, for instance, the probability density of the elution time of a single molecule. In the three-parameter model given in Eq. 2, the scale factor A may be taken as the number of analyte molecules contributing to a peak multiplied by a response factor.

As is known, the functional form of Eq. 2 produces a symmetric line shape (skew, $S$, equal to unity) and, thus, step **511** in the method **150** (FIG. 9) utilizes a Gaussian line shape when the estimated peak skew is in the vicinity of unity, that is when $(1-\varepsilon) \le S \le (1+\varepsilon)$ for some positive quantity $\varepsilon$. In the illustration shown in FIG. 9, the quantity $\varepsilon$ is taken as 0.05, but it could be any other pre-defined positive quantity. A statistical fit may performed within a range of data points established by a pre-defined criterion. For instance, the number of data points to be used in the fit may be calculated by starting with a pre-set number of points, such as 12 points and then adjusting, either increasing or decreasing, the total number of data points based on an initial estimated peak width. Preferably, downward adjustment of the number of points to be used in the fit does not proceed to less than a certain minimum number of points, such as, for instance, five points.

Alternatively, the fit may be mathematically anchored to the three points shown in FIG. 11. Alternatively, the range of the fit may be defined as all points of the peak occurring above the noise threshold. Still further alternatively, the range may be defined via some criterion based on the intensities of the points or their intensities relative to the maximum point 606, or even on criterion based wholly or in part on calculation time. Such choices will depend on the particular implementation of the method, the relative requirements for calculation speed versus accuracy, etc.

If $S>(1+\varepsilon)$, then the data peak is skewed so as to have an elongated tail on the righthand side. This type of peak may be well modeled using either a line shape based on either the Gamma distribution function or on an exponentially modified Gaussian (EMG) distribution function. Examples of peaks that are skewed in this fashion (all of which are synthetically derived Gamma distributions) are shown in FIG. 12. If the peaks in FIG. 12 are taken to be chromatograms, then the abscissa in each case is in the units of time, increasing towards the right.

The general form of the Gamma distribution function, as used herein, is given by:

$$I\left(x; A, x_0, M, r\right) = A\frac{r^M (x - x_0)^{M-1} e^{-r(x-x_0)}}{\Gamma(M)} \quad x \ge x_0 \qquad \text{Eq. 3}$$

in which the dependent and independent variables are $x$ and $I$, respectively, as previously defined, $\Gamma(M)$ is the Gamma function, defined by $\Gamma(M) = \int_0^\infty u^{M-1} e^{-u} du$ and are $A$, $x_0$, $M$ and $r$ are parameters, the values of which are calculated by methods of the present teachings. Note that references often provide this in a "normalized" form (i.e., a probability density function), in which the total area under the curve is unity and which has only three parameters. However, as noted previously herein, the peak area parameter A may be taken as corresponding to the number of analyte molecules contributing to the peak multiplied by a response factor.

The inventors consider that a chromatographic peak of a single analyte exhibiting peak tailing may be modeled by a four-parameter Gamma distribution function, wherein the parameters may be inferred to have relevance with regard to physical interaction between the analyte and the chromatographic column. In this case, the Gamma function may be written as:

$$I\left(t; A, t_0, M, r\right) = A \frac{r^M \left(t - t_0\right)^{M-1} e^{-r\left(t-t_0\right)}}{\Gamma(M)} \quad t \geq t_0 \qquad \text{Eq. 3a}$$

in which $t$ is retention time (the independent variable), $A$ is peak area, $t_0$ is lag time and $M$ is the mixing number. Note that if $M$ is a positive integer then $\Gamma(M) = (M-1)!$ and the distribution function given above reduces to the Erlang distribution. The adjustable parameters in the above are A, $t_0$, $M$ and $r$. FIG. 12 illustrates four different Gamma distribution functions for which the only difference is a change in the value of the mixing parameter, $M$. For curves **702, 704, 706** and **708,** the parameter $M$ is given by $M$=2, $M$=5, $M$=20 and $M$=100, respectively. In the limit of high $M$, the Gamma function approaches the form of a Gaussian function.

The general, four-parameter form of the exponentially modified Gaussian (EMG) distribution, as used in methods according to the present teachings, is given by a function of the form:

$$I\left(x; A, x_0, \sigma^2, \tau\right) = A \int_{-\infty}^{x} \frac{1}{\sigma\sqrt{2\pi}} e^{-(u-x_0)^2 / 2\sigma^2} \frac{1}{\tau} e^{-(x-u)/\tau} du \qquad \text{Eq. 4.}$$
$$\left(x \geq 0; \tau > 0\right)$$

Thus, the EMG distribution used herein is defined as the convolution of an exponential distribution with a Gaussian distribution. In the above Eq. 4, the independent and dependent variables are x and I, as previously defined and the parameters are A, t0, $\sigma$2, and $\tau$. The parameter A is the area under the curve and is proportional to analyte concentration and the parameters t0 and $\sigma$2 are the centroid and variance of the Gaussian function that modifies an exponential decay function. An exponentially-modified Gaussian distribution function of the form of Eq. 4 may be used to model some chromatographic peaks exhibiting peak tailing. In this situation, the general variable x is replaced by the specific variable time t and the parameter x0 is replaced by t0.

FIG. 13 illustrates, in greater detail, various sub-steps that may be included in the step **512** of the method **150** (see FIG. 5 and FIG. 9) within embodiments in accordance with the present teachings. More generally, FIG. 13 outlines an exemplary method for choosing between line shape forms in the modeling and fitting of an asymmetric spectral peak. The method **512** illustrated in FIG. 13 may be entered from step **510** of the method **150** (see FIG. 9).

When method **512** is entered from step **510** (see FIG. 9), the skew, S, is greater than (1+$\epsilon$), because the respective "No" branch has previously been executed in each of steps **509** and **510** (see FIG. 9). For instance, if $\epsilon$ is set to 0.05, then the skew is greater than 1.05. When $S$>(1+$\epsilon$), both the EMG distribution (in the form of Eq. 4) and the Gamma distribution may be fit to the data and one of the two distributions may be selected as a model of better fit on the basis of the squared difference (chi-squared statistic).

From step **808,** the method **512** (FIG. 13) proceeds to step **810.** In these two steps, first one line shape and then an alternative line shape is fitted to the data and a chi-squared statistic is calculated for each. The fit is performed within a range of data points established by a pre-defined criterion. For instance, the number of data points to be used in the fit may be calculated by starting with a pre-set number of points, such as 12 points and then adjusting, either increasing or decreasing, the total number of data points based on an initial estimated peak width. Preferably, downward adjustment of the number of points to be used in the fit does not proceed to less than a certain minimum number of points, such as, for instance, five points.

Alternatively, the fit may be mathematically anchored to the three points shown in FIG. 11. Alternatively, the range may be defined as all points of the peak occurring above the noise threshold. Still further alternatively, the range may be defined via some criterion based on the intensities of the points or their intensities relative to the maximum point **606,** or even on criterion based wholly or in part on calculation time. Such choices will depend on the particular implementation of the method, the relative requirements for calculation speed versus accuracy, etc. Finally, in step **812,** the fit function is chosen as that which yields the lesser chi-squared. The method **512** then outputs the results or exits to step **516** of method **150** (see FIG. 9).

FIGS. 14A-14B are flowcharts that respectively illustrate, in greater detail, alternative sets of sub-steps that may be included in the step **508** of the method **150** (see FIG. 5 and FIG. 9) within embodiments in accordance with the present teachings. More generally, FIGS. 14A and 14B illustrate steps for estimating coordinates of points at a peak maximum and along flanks of the peak at half-height, according to a first exemplary method, method **508a** (FIG. 14A) as well as according to an alternative exemplary method, method **508b** (FIG. 14B) in accordance with the present teachings. Each of the two methods **508a** (FIG. 14A) and **508b** (FIG. 14B) may be entered from step **506** of method **150** (FIG. 9) and may output data or exit to step **509** of method **150**. Upon detection of a peak, the point of maximum intensity (e.g., point **606** of FIG. 11) may be taken as an initial estimate of the peak vertex ($x_m, y_m$) as in step **902** of method **508a.** Alternatively, the sample of maximum intensity and its two nearest neighbors may be fit to a parabola as in step **906** of method **508b,** and then the vertex of the parabola used to provide an estimate of the interpolated peak vertex (which in general does not exactly coincide with a data point of a chromatogram). Next, in either step **904** of method **508a** or step **908** of method **508b,** the left and right half maxima of the detected peak (e.g., points **604** and **608,** respectively, of FIG. 11) are estimated by examining the sample values to the left and right (respectively), scanning outward from the peak vertex until encountering a value that is less than one-half the interpolated maximum value. Interpolated values of the left and right half-maxima are determined by fitting a line to sample points whose intensities lie above and below one-half the maximum intensity and finding the $x$-axis coordinate (either $x_L$ or $x_R$-see FIG. 11) of the point on the line that passes through the half-maximum intensity. Then, the estimated peak skew, $S$, is calculated as $S=(x_R-x_m)/(x_m-x_L)$.

Returning, once again, to the method **48** as shown in FIG. 5, it is noted that, after all peaks have been fit in step **150,** the next optional step, step **170** comprises refinement of the initial parameter estimates for multiple detected chromatographic peaks. Refinement comprises exploring the space of $N$ parameters (the total number of parameters across all peaks, i.e. 4 for each Gamma/EMG and 3 for each Gaussian) to find the set of values that minimizes the sum of squared differences between the observed and model chromatogram. Preferably, the squared difference may be calculated with respect to the portion of the chromatogram comprising multiple or overlapped peaks. It may also be calculated with respect to the entire chromatogram. The model chromatogram is calculated by summing the contribution of all peaks estimated in the previous stage. The overall complexity of the refinement can be greatly reduced by partitioning the chromatogram into regions that are defined by overlaps between the detected peaks. In the simplest case, none of the peaks overlap, and the parameters for each individual peak can be estimated separately.

The refinement process continues until a halting condition is reached. The halting condition can be specified in terms of a fixed number of iterations, a computational time limit, a threshold on the magnitude of the first-derivative vector (which is ideally zero at convergence), and/or a threshold on the magnitude of the change in the magnitude of the parameter vector. Preferably, there may also be a "safety valve" limit on the number of iterations to guard against non-convergence to a solution. As is the case for other parameters and conditions of methods of the present teachings, this halting condition is chosen during algorithm design and development and not exposed to the user, in order to preserve the automatic nature of the processing. At the end of refinement, the set of values of each peak area along with a time identifier (either the centroid or the intensity maximum) is returned. The entire process is fully automated with no user intervention required.

*Section 3. Application to Three-Variable LC-MS Data*

The foregoing description has mainly dealt with characterizing peaks in spectral data comprising just a single independent variable (i.e., a time-related independent variable for XIC data). For example, several such schematic extracted ion chromatograms are illustrated in FIG. 15 by dotted lines residing at respective mass-to-charge values indicated by sections **m1, m2, m3, m4, m5, m6, m7, m8, m9** and **m10.** Subsequent to execution of the methods discussed in Section 2 above, each such XIC is defined by the set of synthetic peaks calculated by those methods. The hypothetical synthetic extracted ion chromatograms schematically shown in FIG. 15 illustrate elution of various ionized chemical constituents at or near various retention times **rt1, rt2** and **rt3.**

The set of extracted ion chromatograms indicated by sections m1-m10 in FIG. 15 could be algebraically summed so as to yield a reconstructed total ion chromatogram (not shown). Likewise, the synthetic peak intensities provided by the peak detection and fitting routines described above could be projected onto the time sections shown at times rt1, rt2 and rt3 in order to generate reconstructed mass spectra (reconstructed "scans"). Reconstructed mass spectra are illustrated by the solid-line curves in FIG. 15. In accordance with the present teachings the reconstructed scans are generated by including all masses that produce a chromatographic peak at the time corresponding to the scan, lie within the linewidth of said peak, and were collected under identical scan filters. Thus, every ion present in a reconstructed scan is known to contribute to a chromatographic peak, whose apex is nearby but not necessarily at the time of the scan.

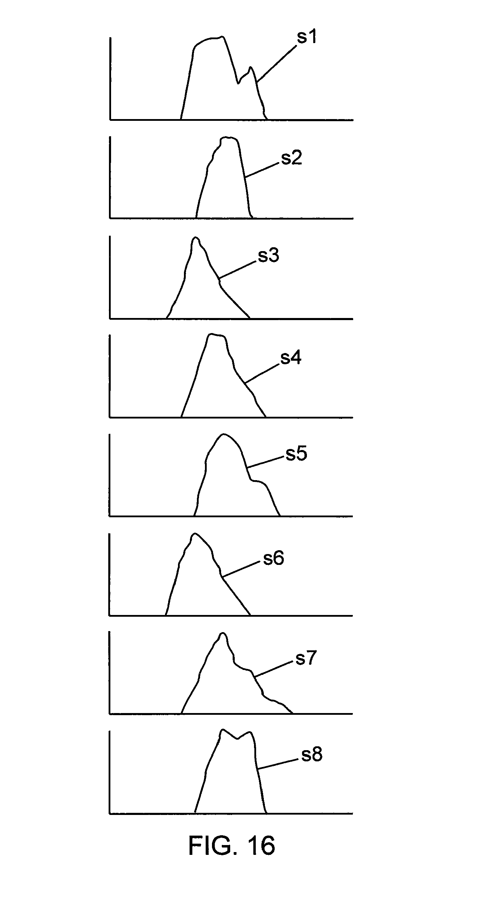

3.1. Line Shape Reproduction by Parameterless Peak Detection Methods. The extracted ion chromatogram (XIC) peak shapes for components that elute at similar times are not all the same, neither are they all different. Figure 16 shows results from a typical situation, in which the peak shapes in various extracted ion chromatograms fall into several groups of patterns indicated by the peak profiles **s1-s8.** The data from which these profiles were generated was obtained using an instrumental system similar to that shown in FIG. 2 and were obtained during the 6-second elution of a single mass chromatographic peak (e.g., a total ion chromatogram peak) of a 500 nM solution of the drug Buspirone.

The profiles **s1-s8** correspond to different respective *m/z* ranges obtained from all-ions fragment data.

Comparison of the illustrated XIC peak profiles in FIG. 15 illustrates how mass spectral peak profiles relating to elution of different isotopes of a single type of molecule (a molecule with a particular atomic composition and structure) may be expected to all have similar elution profiles. Since the chemistry and physics that determine the chromatographic peak shape are unique for each molecule and cease when the molecule exits the column, one can expect that XICs having similar shapes may be related. In a similar manner, profiles relating to elution of different types of molecules may be expected to have different respective shapes, these shape differences resulting from differing interaction of each respective eluate with a particular chromatographic column. Although the various eluate peak shapes may be specific to a particular instrument, these time-profiles are expected to carry over to all of the mass spectral peaks - including those peaks resulting from various isotopes - that result from ionization of a particular eluate.

As an example of the above considerations, hypothetical peaks **p2, p3** and **p4** of FIG. 15 each exhibit an XIC time profile similar to profile **s1** of FIG. 16 and all such profiles occur simultaneously. Thus, these peaks will have strong correlation scores in a subsequent analysis (discussed below) and may be noted as candidates for isotope distribution analysis or modeling. A stronger statement can be made that peaks that exhibit different shapes in their respective XICs are not related as different isotope compositions of the same chemical molecule. For instance, the XICs including peaks **p5** and **p6** of FIG. 15 exhibit a different time profile that, although highly correlated between peaks **p5** and **p6 ,** is dissimilar to the profiles of peaks **p2, p3** and **p4.** Thus, peaks **p5** and **p6** may result from co-elution of an eluate together with the eluate that gives rise to **p2, p3** and **p4.** Peak **p1** does not correlate well with any of peaks, **p2-p6,** having a different time-profile than any of the peaks **p2-p6** and also offset in time to time **rt2.** An isolated peak, such as peak **p1,** may result from background "noise" which is not related to any of the analytes of interest. At time **rt3,** a different compound may elute in the same LC/MS analysis. In this example, peaks **p8, p9** and **p10** all correlate well with one another as each of them exhibits a peak shape profile that is similar to profile **s2** of FIG. 16 and whose center of mass is centered at time **rt3.** Thus, these peaks may be noted as candidates for isotope distribution analysis or modeling. Peak **p7,** by comparison, has a low correlation score with respect to any of peaks **p8, p9** and **p10** and may be assigned to co-elution of a different compound. By using Parameterless Peak Detection (PPD) techniques, as described in Section 2 herein, to characterize the peak shape, small differences in shape can be encoded in a correlation vector (described in more detail following). This can be enhanced by additional smoothing after the peak is detected (but not before, since prior smoothing can smooth a noise spike into a peak). Step **59** of method **40** (FIG. 4A) is the cross-correlation step which is described in more detail in the following section.

3.2. Cross Correlation Calculations. Overall cross-correlation scores (CCS) in accordance with the present teachings are calculated (i.e., in step **59** of method **40)** according to the following strategy. For each mass in the experimental data that is found to form a chromatographic peak by PPD as described in Section 2, the cross correlation of every mass with every other mass is computed. In the present context, the term "peak" refers simply to masses that have non-zero intensity values for several contiguous or nearly contiguous scans (for example, the scans at times **rt1**, **rt2, rt3** and **rt4** illustrated in FIG. 15) of the same type. Each cross-correlation score may be calculated as a weighted average of a peak shape correlation score (calculated in terms of a time-versus-intensity for each mass that forms a recognized peak) and an optional peak width correlation score as described below. If a calculated overall correlation score is such that a match between different peaks of an isotopic distribution is recognized, then these peaks are assigned as having been ionized from the same molecule. According to a slightly different strategy, a calculated overall correlation score might be used to recognize a set of peaks that result from ionization of a same molecule. Then, the spacing and intensity distribution of these peaks may be used to identify related peaks belonging to an isotopic distribution. Peaks having low correlation are not assigned as belonging to an isotopic distribution relating to ionization of a single type of molecule.

Methods in accordance with the present teachings use a trailing retention time window to calculate peak-shape cross correlations. The methods make use of a numerical array including mass, intensity, and scan number values for every mass that forms a chromatographic peak. As described in Section 2, Parameterless Peak Detection (PPD) is used to calculate a peak shape for each mass component. This shape may be a simple Gaussian or Gamma function peak, or it may be a sum of many Gaussian or Gamma function shapes, the details of which are stored in a peak parameter list. Once the component peak shape has been characterized by an analytical function (which may be a sum of simple functions), the problem of calculating a vector product ("dot product") correlation is greatly simplified. These cross correlations are normalized by also calculating, and dividing by, the autocorrelation values. Consequently, the peak shape correlation (PSC) between two peak profiles, p1 and p2 (denoted, functionally as $p1(t)$ and $p2(t)$, where *t* represents a time variable, may be calculated as

$$PSC(p1,p2) = \frac{\sum\limits_{j=j\min}^{j=j\max}\left[p1(t_j)\times p2(t_j)\right]}{\left\{\sum\limits_{j=j\min}^{j=j\max} p1(t_j)^2\right\}^{1/2}\left\{\sum\limits_{j=j\min}^{j=j\max} p2(t_j)^2\right\}^{1/2}} \qquad \text{Eq. 5}$$

in which the time axis is considered as divided into equal width segments, thus defining indexed time points, $t_j$, ranging from a practically defined lower time bound, $t_{j\,min}$, to a practically defined upper time bound, $t_{j\,max}$. Accordingly, the quantity PSC can theoretically have a range of 1 (perfect correlation) to -1 (perfect anti-correlation), but since negative going chromatographic peaks are not detected by PPD (by design) the lower limit is effectively zero. For example, the lower and upper time bounds, $t_{j\,min}$, and, $t_{j\,max}$, may be set in relation to each peak that may represent one ion of an isotopic distribution of ions produced by ionization of a single type of molecule. In such a case, the time values are chosen so as to sample intensities a fixed number of times (for instance, between roughly seven and fifteen times, such as eleven times) across the width of a peak. The mass peaks to be tested for correlation with the chosen mass peak then use the same time points. This means that if these masses form a peak at markedly different times, the intensities will be essentially zero. Partially overlapped peaks will have some zero terms.

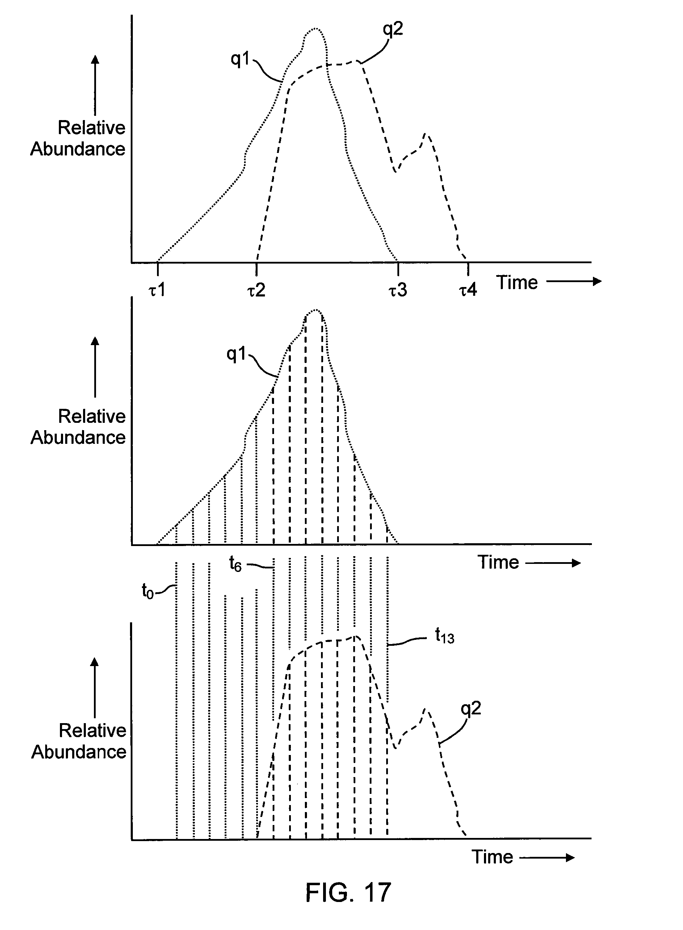

FIG. 17 graphically illustrates calculation of a dot product cross-correlation score in this fashion. In FIG 17, two hypothetical XIC peak profiles **q1** and **q2** are shown. Peak **q1** has appreciable intensity above baseline only between time points $\tau1$ and $\tau3$ and peak **q2** has appreciable intensity only between time points $\tau2$ and $\tau4$. As discussed above, to calculate the dot-product cross correlation score between these two peaks, the retention time axis may be considered as being divided into several equal segments between time points $\tau1$ and $\tau3$, thereby defining, in this example, indexed time points $t_j$ where ($0 \le j \le 13$). The two peak profiles are shown separately in the lowermost two graphs of FIG. 17 in association with vertical lines representing the various indexed time points along the retention time axis. In this representation, peak **q2** only has appreciable intensity between the points $t_6$ and $t_{(13)}$. Thus, in this example, the peak shape correlation is given by

$$PSC(q1,q2) \quad = \quad \frac{\sum\limits_{j=0}^{j=13}\left[q1(t_j)\times q2(t_j)\right]}{\left\{\sum\limits_{j=0}^{j=13} q1(t_j)^2\right\}^{1/2}\left\{\sum\limits_{j=0}^{j=13} q2(t_j)^2\right\}^{1/2}}$$

Under such a calculation, the cross-correlation score, as calculated above, for the peaks **q1** and **q2** illustrated in FIG. 17 would be a positive number because the peaks partially overlap, but would be below a threshold score for recognizing a peak match, since the peaks have different shapes. The cross-correlation score for a peak with itself or with a scaled version of itself is unity, as would be expected for ions resulting from ionization of the same type of molecule. Note from FIG. 15 that, by this measure, any pair of peaks chosen from the peaks **p2, p3** and **p4** would yield a high cross-correlation score even though these peaks have different magnitudes. By contrast, the cross-correlation score between the peaks **p7** and **p9** illustrated in FIG. 15 would be lower, even though these latter two peaks are of roughly equal magnitude, because the time profiles of these peaks have different shapes.

If it is desired to also use a peak width correlation, this may be calculated using the absolute peak widths as determined by PPD on the XIC peak shapes. Accordingly, an optional peak width correlation, $PWC_{(p1,p2)}$, between peaks **p1** and **p2** may be calculated by

$$PWC_{(p1,p2)} = 1 - B \left| \text{width}_{p1} - \text{width}_{p2} \right| \qquad \text{Eq. 6}$$

in which $B$ is the inverse of the maximum of $\text{width}_{p1}$ and $\text{width}_{p2}$ and the vertical bars represent the mathematical absolute value operation.

The cross-correlation score, as shown in step **46** of method **40** (FIG. 4A) is calculated using the peak-shape correlation score, PSC, and optionally combining it together with the peak width correlation score, PWC, as a weighted average. Accordingly, the overall correlation score, $CCS_{(p1,p2)}$, is given by

$$CCS_{(p1,p2)} = \{ X[PSC_{(p1,p2)}] + Z[PWC_{(p1,p2)}] \} / \{X + Z\} \qquad \text{Eq. 7}$$

in which $X$ and $Z$ are weighting factors. Thus, the overall score, CCS, ranges from 1.0 (perfect match) down to 0.0 (no match). Peak matches are recognized when a correlation exceeds a certain pre-defined threshold value. Experimentally, it is observed that limiting recognized matches to scores to those above 0.90 provides excellent results.

It may be noted that since the values of the mass for each scan in the group of scans and intensities that form a chromatographic peak are known, there is the opportunity to do some trend analysis of the values. Consequently it should be possible to correct for linear drifts in the mass values, or a systematic mass shift due to ion intensity.

*Section 4. Examples*

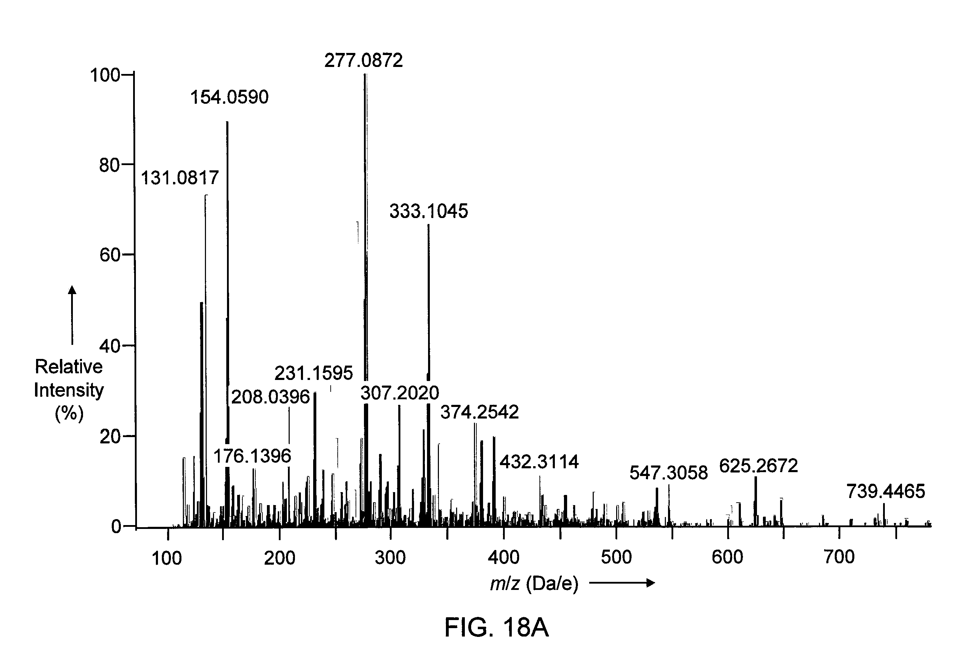

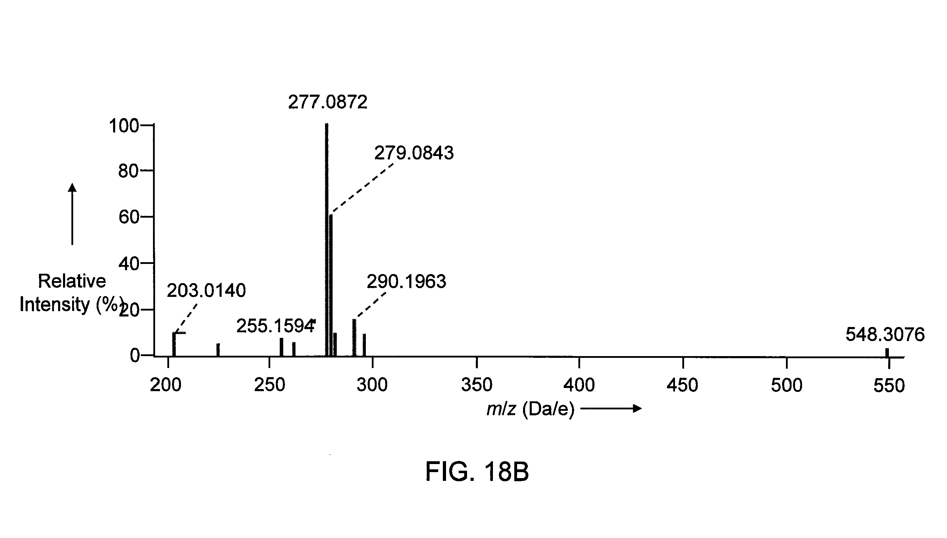

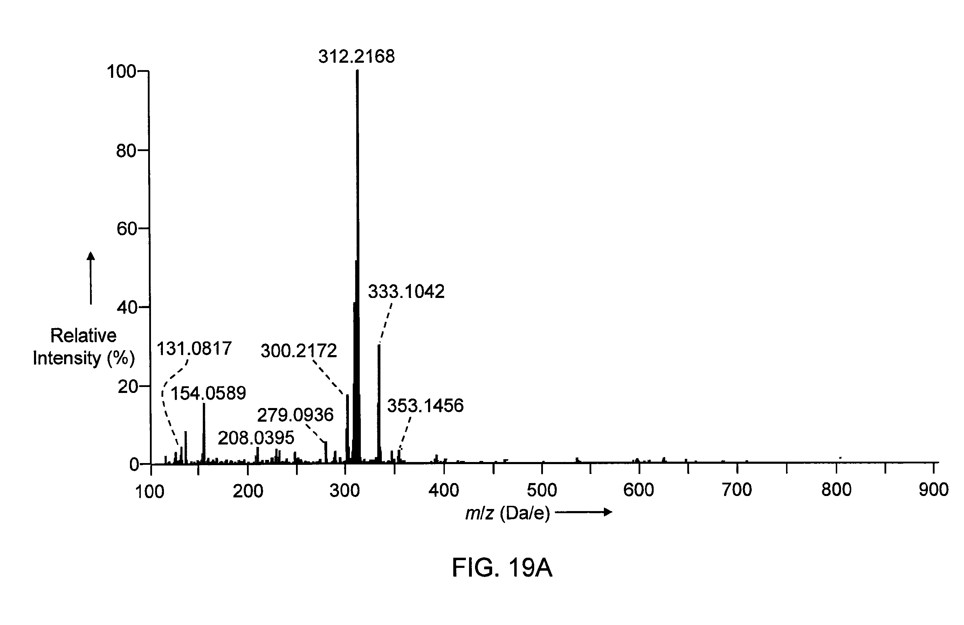

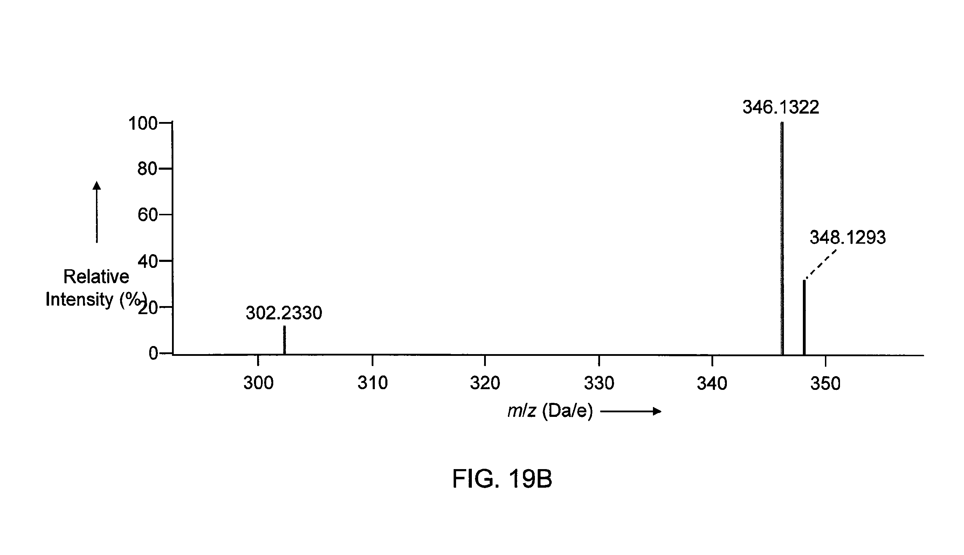

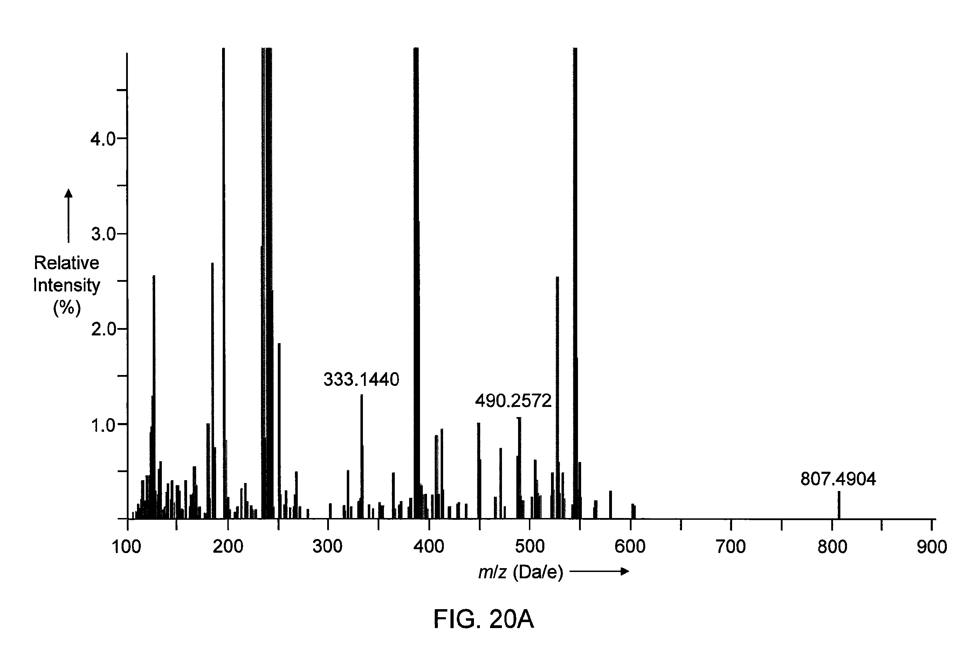

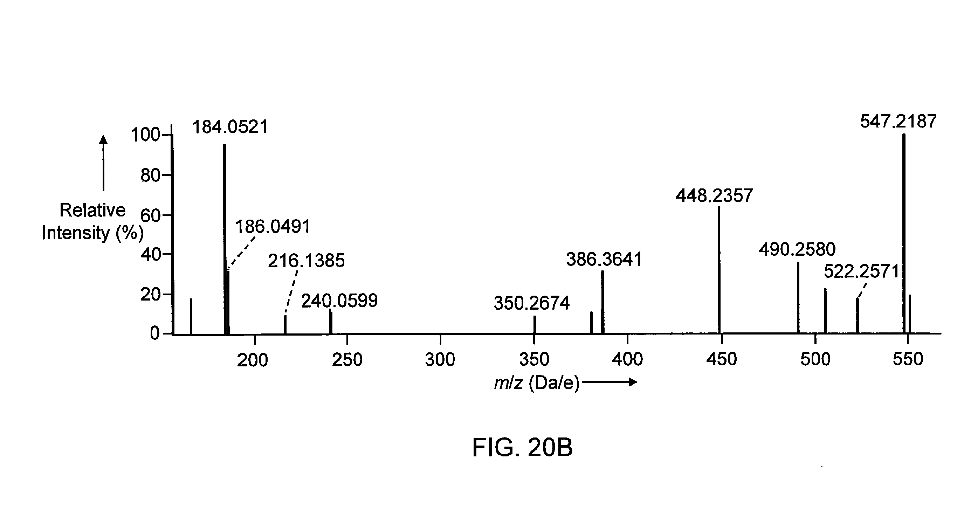

FIGS. 18-20 provide examples of the practice of methods in accordance with the present teachings. FIG. 18A and FIG. 19A are mass spectral scans of two different eluates acquired during an LC/MS analysis of a single diluted urine sample. FIG. 18B and FIG. 19B are, respectively, calculated subsets of the mass spectral peaks shown FIG. 18A and FIG. 18B determined by performing correlation analyses in accordance with the present teachings. In other words, FIGS. 18B and 19B show peaks of ions whose time-profiles are correlated with one another according to the above described methods. It should be noted that these correlation analyses were performed after removal of peaks obviously related to $^{13}C$ isotope distributions. The reconstructed spectra would be much more complex if these peaks arising from isotope distributions relating to the major element carbon were to be retained. In FIG. 18B, the triplet at 277/279/281 is consistent with a fragment containing 2 chlorine atoms. In FIG. 19B, the doublet at 346/348 is consistent with a different fragment containing a single chlorine atom. Another example is illustrated in FIGS. 20A-20B, which is a mass spectral scan of an eluate from a diluted bile sample. In these spectra, a somewhat less-intense spectral profile exhibits a chlorine isotope pattern at masses 184/186. Comparing the experimental data with a simulation, the data of FIG. 20B are very well fit by a simulation of $C_9H_{11}ONCl$.

*Conclusions*

The novel methods taught herein are able to identify $m/z$ values in a full-MS scan that are related by elution lineshape. In many cases, these related $m/z$ values come from isotope multiplets. The novel methods according to the present teachings assign discovered multiplets to elemental isotopes and enable determinations of element abundances, using high correlation scores to give confidence that the assignments are real and not associations of chance. These methods are useable on any LS/MS or GC/MS instrument. High mass accuracy, while helpful, is not a requirement. The use of extracted ion chromatograms (XICs) yields virtually-noise-free spectral data which may be reliably and reproducibly employed for automated peak identification and peak cross correlation calculations. The XICs may also be used for automated isotope pattern recognition, thereby yielding calculated results with a high degree of confidence.

The newly invented methods described herein have no user-adjustable parameters, and can be run automatically in a post-acquisition step, or implemented in firmware and the new, simplified output files created at acquisition time. By using fitted parametric functions to describe the data, problems of normalization and time shifting of data points are totally eliminated, and all peaks may easily be characterized by an array of N values. This greatly simplifies the calculations of vector dot products between various peak shapes as used in the methods. Although the described methods are somewhat computationally intensive, they are nonetheless able to process data faster than it is acquired, and so can be done in real time, so as to make automated real-time decisions about the course of subsequent mass spectral scans on a single sample or during a single chromatographic separation. Such real-time (or near-real-time) decision making processes require data buffering since chromatographic peaks are searched for in a moving window of time.

Computer instructions according to any of the methods described above may be supplied as a computer program or as a computer readable medium, such as disk storage (fixed magnetic disk, floppy disk drive, optical disk drive, magneto-optical disk drive), magnetic tape, or non-volatile memory device containing program instructions readable by an electronic processor, such computer program product or products and storage devices themselves being aspects of the present teachings.

The discussion included in this application is intended to serve as a basic description. Although the invention has been described in accordance with the various embodiments shown and described, one of ordinary skill in the art will readily recognize that there could be variations to the embodiments and those variations would be within the essence and scope of the present invention. The reader should be aware that the specific discussion may not explicitly describe all embodiments possible; many alternatives are implicit. Accordingly, many modifications may be made by one of ordinary skill in the art without departing from the scope and essence of the invention. Neither the description nor the terminology is intended to limit the scope of the invention.

**Claims**

1. A method for automatically identifying groups of related peaks generated in a chromatography - mass spectrometry experiment, the method including the steps of automatically choosing a time window defining a region of interest for mass spectral data generated by the experiment; automatically constructing a plurality of extracted ion chromatograms (XICs) for mass spectral peaks observed within the region of interest; and automatically detecting and characterizing chromatogram peaks within each XIC and automatically generating synthetic analytical fit peaks thereof; the method **characterized by** the steps of:

   automatically discarding a subset of the synthetic analytical peaks which do not satisfy noise reduction rules;
   automatically performing a respective cross-correlation score calculation between each pair of synthetic analytical fit peaks; and
   automatically identifying groups of correlated peaks in at least one mass spectrum within the region of interest.

2. A method as recited in claim 1, further comprising reporting the indentified groups of correlated peaks to a user.

3. A method as recited in claim 1, wherein the step of automatically identifying groups of correlated peaks in at least one mass spectrum within the region of interest includes automatically identifying at least one group of peaks that correspond to an isotopic distribution.

4. A method as recited in claim 4, further **characterized by** the further step of indentifying a chemical composition of an ionic species based upon the automatic identification of the at least one group of peaks that corresponds to an isotopic distribution.

5. A method as recited in claim 1, further **characterized in that** the step of automatically identifying groups of correlated peaks in at least one mass spectrum within the region of interest includes automatically identifying a first group of peaks that corresponds to a first isotopic distribution and a second group of peaks that corresponds to a second isotopic distribution, wherein the first and second groups of peaks are associated with elution of a first and a second analyte, respectively, wherein the first and second analytes co-elute.

6. An apparatus comprising a chromatograph for providing a stream of at least partially separated chemical substances; a mass spectrometer fluidically coupled to the chromatograph for generating and analyzing a plurality of types of ions, each type of ion comprising a different mass-to-charge ($m/z$) ratio resulting from ionization of each of the chemical substances; a detector for detecting abundance data for each type of ion; a programmable electronic processor electrically coupled to the mass spectrometer, the apparatus **characterized by**:

   a computer readable medium electrically coupled to the programmable electronic processor and having instructions thereon operable to cause the programmable processor to:

   receive the abundance data for each of the types of ions from the detector;
   automatically detect and characterize chromatogram peaks as a function of time for each of a plurality of $m/z$ ratio ranges of the abundance data;
   automatically generate synthetic analytical fit peaks to the detected chromatogram peaks;
   automatically discard a subset of the synthetic analytical fit peaks which do not satisfy noise reduction rules;

automatically perform a respective cross-correlation score calculation between each pair of synthetic analytical fit peaks; and

automatically identify groups of correlated peaks in at least one mass spectrum within the region of interest.

7. An apparatus as recited in claim 6, wherein the computer readable medium further comprises instructions operable to cause the programmable processor to report the indentified groups of correlated peaks to a user.

8. An apparatus as recited in claim 6, wherein the computer readable medium further comprises instructions operable to cause the programmable processor to automatically identify at least one group of peaks that correspond to an isotopic distribution.

9. An apparatus as recited in claim 8, wherein the computer readable medium further comprises instructions operable to cause the programmable processor to automatically identify a chemical composition of an ionic species based upon the automatic identification of the at least one group of peaks that corresponds to an isotopic distribution.

<u>10</u>

FIG. 1

<u>15</u>

FIG. 2
(Prior Art)

FIG. 3

FIG. 4A

_40_

from Step 49,
FIG. 4A

Current peak = first XIC peak — 51

Area / intensity
rules satisfied? — 52

N → Discard peak
from
candidates — 53

Y

Retain peak for cross correlation — 54

Subtract peak area — 55

Note peak as having
been considered — 56

More peaks
in XIC? — 57

N → to Step 44,
FIG. 4A

Y

Current peak =
next XIC peak — 58

FIG. 4B

48

Detect and characterize peaks using PPD

120

Remove baseline; estimate noise (FIG. 6)

150

Detect peaks (FIG. 9)

170

Optionally, simultaneously optimizing overlapping peaks

FIG. 5

300

302

Area under curve

Baseline polynomial order

FIG. 7

400

402

Intensity

Time

FIG. 8

FIG. 6

150

502 Locate most intense peak

506 Set initial "difference chromatogram"

508 Make initial estimates of coordinates

509 $S<(1-\varepsilon)$ ?

510 $S \leq (1+\varepsilon)$ ?

515 Model as sum of Gaussians

511 Model as single Gaussian

512 Model with Gamma or EMG line shape

516 Subtract synthetic best-fit peak

518 Adjust peak parameters (if necessary)

520 Subtract adjusted fit peak

522 Add parameters of adjusted peak to list

523 Calculate RMS of difference

526 $I_j<(\text{RMS} \times \xi)$ ?

527 Exit

528 Locate most intense peak

530 Set current greatest peak

FIG. 9

FIG. 10A

540

Relative
Abundance

a2

a1

Time (min)

FIG. 10B

542

FIG. 10C

b1

b2

b3

544

546

$$S = (x_R - x_m)/(x_m - x_L)$$

**FIG. 11**

**FIG. 12**

Fit current greatest peak with either Gamma or EMG line shape

### 512

```
┌ ─ ─ ─ ─ ─ ─ ─ ─ ─ ─ ─ ┐
    From Step 510
└ ─ ─ ─ ─ ─ ─ ─ ─ ─ ─ ─ ┘
            │
            ▼                    808
┌─────────────────────────┐
│   Fit Gamma P.D.F.;      │
│   Calculate chi-squared  │
└─────────────────────────┘
            │
    810     ▼
┌─────────────────────────┐
│  Fit EMG function;       │
│  Calculate chi-squared   │
└─────────────────────────┘
            │
            ▼                    812
┌─────────────────────────┐
│  Choose fit function     │
│  yielding lesser         │
│  chi-squared             │
└─────────────────────────┘
            │
            ▼
┌ ─ ─ ─ ─ ─ ─ ─ ─ ─ ─ ─ ┐
   Exit To Step 516
└ ─ ─ ─ ─ ─ ─ ─ ─ ─ ─ ─ ┘
```

## FIG. 13

### 508a
                                                    902

```
┌ ─ ─ ─ ─ ┐          ┌─────────────────────────┐          ┌ ─ ─ ─ ─ ┐
 From Step  ─ ─ ─ ▶  │ Locate peak vertex,      │  ─ ─ ┐    To Step
   506     │          │        (xₘ,yₘ)           │      │   │   509
└ ─ ─ ─ ─ ┘          └─────────────────────────┘      │   └ ─ ─ ─ ─ ┘
                              │                         │
              904             ▼                         │
             ┌─────────────────────────────┐            │
             │ Find interpolated (xₗ, yₘ/2) │  ─ ─ ─ ─ ┘
             │ and (xᵣ, yₘ/2); set          │
             │ S=(xᵣ-xₘ)/(xₘ-xₗ)            │
             └─────────────────────────────┘
```

Find interpolated $(x_L, y_m/2)$ and $(x_R, y_m/2)$; set $S=(x_R-x_m)/(x_m-x_L)$

## FIG. 14A

### 508b
                                                    902

```
┌ ─ ─ ─ ─ ┐          ┌─────────────────────────┐          ┌ ─ ─ ─ ─ ┐
 From Step  ─ ─ ─ ▶  │ Estimate peak vertex     │  ─ ─ ┐    To Step
   506     │          │     with parabola        │      │   │   509
└ ─ ─ ─ ─ ┘          └─────────────────────────┘      │   └ ─ ─ ─ ─ ┘
                              │                         │
              904             ▼                         │
             ┌─────────────────────────────┐            │
             │ Find interpolated (xₗ, yₘ/2) │  ─ ─ ─ ─ ┘
             │ and (xᵣ, yₘ/2); set          │
             │ S=(xᵣ-xₘ)/(xₘ-xₗ)            │
             └─────────────────────────────┘
```

Find interpolated $(x_L, y_m/2)$ and $(x_R, y_m/2)$; set $S=(x_R-x_m)/(x_m-x_L)$

## FIG. 14B

FIG. 15

FIG. 16

FIG. 17

FIG. 18A

FIG. 18B

FIG. 19A

EP 2 594 936 A2

FIG. 19B

FIG. 20A

EP 2 594 936 A2

FIG. 20B

EP 2 594 936 A2

## REFERENCES CITED IN THE DESCRIPTION

**Patent documents cited in the description**

- US 20100100336 A1
- WO 02078046 A
- US 5886346 A
- US 6872938 B